# EUROPEAN PATENT APPLICATION

(11) **EP 4 368 203 A1**
(43) Date of publication of application: **15.05.2024**
(21) Application number: 22836906.2
(22) Date of filing: 05.07.2022
(51) Int. Cl.: A61K 39/395, A61K 47/64, A61K 38/16, A61K 48/00, C12N 15/113, C12N 15/864

(54) **CONSTRUCTION AND USE OF ANTI-VEGF ANTIBODY IN-VIVO EXPRESSION SYSTEM**

(30) Priority: 05.07.2021 CN 202110757277
(71) Applicant: Wuhan Neurophth Biotechnology Limited Company, Wuhan, Hubei 430060 (CN)
(72) Inventor: LI, Bin, Wuhan, Hubei 430060 (CN); LI, Qiutang, Wuhan, Hubei 430060 (CN); XIAO, Lu, Wuhan, Hubei 430060 (CN); ZHANG, Shanshan, Wuhan, Hubei 430060 (CN)
(74) Representative: Cooley (UK) LLP
(86) International application number: PCT/CN2022/103897
(87) International publication number: WO 2023/280157

(57) **Abstract**

The present invention relates to the field of genetic engineering, and particularly to the construction of a system comprising a gene fragment expressing an anti-VEGF antibody, and use thereof in the treatment of neovascularization, including age-related macular degeneration, and diabetic macular edema, etc. The system has the advantages of stable expression, long-lasting effectiveness, and low risk of tissue damage.

## Description

### Cross-reference to related application

The present application claims the priority to Chinese Patent Application No. 202110757277.9, the disclosure of which in incorporated herein by reference in its entirety.

### Technical field

The present invention relates to the technical field of genetic engineering, and in particular to the construction and use of a system comprising a gene fragment expressing an anti-VEGF antibody. Specifically, the system is an approach to delivering a macromolecular drug gene by a gene therapy vector for in vivo expression to directly exert a biological function. This approach eliminates the complex process of production and purification of traditional macromolecule drugs, and allows for continuous and stable expression of a therapeutically effective dose of a bioactive macromolecule in the body.

### Background

Age-related macular degeneration (AMD) and diabetic retinopathy (DR) are two major causes of blindness. Age-related macular degeneration (AMD) is a disease that causes the loss of central vision, involving the macula. The macula is located at the center of the retina, and is a light-sensitive tissue that lines the back of the eye and provides clear central vision. Retina is a thin layer of tissue that lines the back of the eye and receives light. The retina converts light or images into electrical impulses, and then passes these impulses or nerve signals to the brain to form vision. The damage caused by AMD significantly affects the eye's ability to convert these signals into images. Yellow deposits (drusen) is formed under the retina, causing distortion and blurring of vision. Over time, the deposits increase in size and amount. This causes blood vessels to grow under the retina (angiogenesis), leaking blood and damaging the retina. Peripheral vision (side vision) may not be affected, but in advanced AMD, the ability to see directly ahead is lost. There are two types of age-related macular degeneration (AMD): dry and wet. In dry age-related macular degeneration, the retinal pigment epithelium, Bruch's membrane, choriocapillaris and other layers gradually shrink and degenerate. Once dry age-related macular degeneration progresses to an advanced stage, no treatment can prevent vision loss and it has no therapeutic significance.

The AMD targeted by the present invention is wet age-related macular degeneration (wAMD). The reason is that damage to Bruch's membrane caused by drusen induces new blood vessels from choroidal capillaries to grow to the outer layer (i.e. choroidal neovascularization (CNV). The new blood vessels are accompanied by fibroblast proliferation, and can destroy choroidal capillaries, Bruch's membrane, pigment epithelial cells and photoreceptor cells, causing severe vision loss.

Wet age-related macular degeneration is currently treated by laser therapy, photodynamic therapy or injection therapy. In the laser therapy, a high-energy beam of light is directed at new blood vessels to destroy them and prevent further vision loss. Laser treatment can also damage some peripheral healthy tissues, so only a small proportion of patients with wet AMD are suitable candidates for laser treatment. If the leaking blood vessel is located away from the fovea, laser treatment is suitable. In the photodynamic therapy (PDT), verteporfin is a second-generation porphyrin photosensitizer that can be activated by light (with a wavelength of 689 nm). Through non-thermal laser irradiation, reactive oxygen species is generated and abnormal blood vessels are occluded, thereby stopping the leakage of blood vessels and preserving vision. Verteporfin is injected into the veins of the arm and the drug "sticks" to the surface of the new blood vessels, which are then activated by irradiating a beam of light into the lesion site in the patient's eye for about 90 sec. Unlike common laser treatments, this drug does not destroy the peripheral healthy tissue. However, because the drug is activated by light, skin and eye exposure to the sunlight and bright indoor light must be avoided for five days after treatment. The injection therapy is an anti-vascular endothelial cell growth factor therapy. The vascular endothelial growth factor (VEGF) in the eyes of patients with wet AMD is abnormally increased, promoting the growth of abnormal new blood vessels. Drug treatments such as ranibizumab can block the action of this growth factor. This therapy is helpful for delaying the vision loss caused by AMD, and improves the vision under certain circumstances.

However, the risk of recurrence of new blood vessels after laser treatment is very high. The photodynamic therapy can slow down the rate of vision loss, but it cannot stop the progress of vision loss or restore vision that has been lost due to late-stage AMD. Repeated treatments are usually required. The injection treatment also currently requires multiple injections.

Diabetic retinopathy (DR) is the most important manifestation of diabetic microvascular disease. It is a fundus pathological change with specific changes and one of the serious complications of diabetes. Clinically, depending on the presence of retinal neovascularization as a sign, the diabetic retinopathy without retinal neovascularization is called non-proliferative diabetic retinopathy (NPDR) (or simple type or background type), and the diabetic retinopathy with retinal neovascularization is called proliferative diabetic retinopathy (PDR). The fundus manifestations of non-proliferative diabetic retinopathy include: retinal vein dilation, microaneurysm, deep and superficial hemorrhage, hard exudation, cotton wool spots, retinal edema, cystoid macular edema formed by long-term macular edema, and significant decrease in vision. In the proliferative diabetic retinopathy, the damage is further aggravated, and a large area of capillaries is occlusive and ischemic, resulting in retinal neovascularization. The new blood vessels then grow from the retinal surface into the space between the internal limiting membrane and the posterior limiting membrane, forming a fibrovascular membrane. New blood vessels are prone to rupture and bleeding, and large vitreous hemorrhage and organization can lead to tractional retinal detachment. The vascular growth factors produced by the retina in the ischemic area enter the anterior chamber through the vitreous body, causing the formation of new blood vessels in the iris and chamber angle, and eventually leading to secondary angle-closure glaucoma, that is, neovascular glaucoma, and blindness.

Diabetic macular edema (DMO) is a type of diabetic retinopathy, which is retinal thickening or hard exudate deposits caused by the accumulation of extracellular fluid within one optic disc diameter of the fovea caused by diabetes. Over the past three decades, early screening and prevention of proliferative diabetic retinopathy has reduced the incidence year by year. Therefore, DMO has now become the main cause of blindness in DM patients. The most important structural change in DMO is the destruction of the blood-retinal barrier. The DM environment causes the destruction of some tight junction proteins, ultimately leading to vascular leakage and changes in its permeability. The most important molecular mediator responsible for this change is vascular endothelial growth factor (VEGF) synthesized by the retina.

Among the treatment methods for DR, drug treatments such as calcium dobesilate, difamin, and mecobain are mainly used for non-proliferative diabetic retinopathy.

Laser photocoagulation destroys diseased tissue through its damaging effect, and changes it from oxygen consuming to non-oxygen consuming, thereby allowing a healthy retinal tissue to have good oxygen supply and promoting the gradual shrinkage of new blood vessels. However, the purpose of photocoagulation is to preserve the vision as much as possible and prevent the disease from deterioration. It works by destroying the abnormal retina, preventing the formation of new blood vessels and the leakage of fluid. However, the disease is still progressing, abnormal new blood vessels and leakage continuously occur, and re-treatment is required.

Vitrectomy surgery is a very effective method to treat proliferative diabetic retinopathy. It clears the refractive stroma, removes accumulated blood and decomposed substances, cuts and sucks out the organized membrane, eliminates the scaffold on which fibrous tissue grows, and loosens the traction on the retina and injects liquid and/or gas to restore the normal anatomical relationship of the retina and keep the eyeball intact, and causes intraocular photocoagulation. The success rate of early surgery can reach 90% or higher, and some patients can restore their vision and control the development of the disease. However, once the disease reaches an advanced stage, surgery is difficult to succeed. Ocular neovascularization is the main pathological sign of age-related macular degeneration (AMD) and diabetic retinopathy (DR), and vascular endothelial growth factor (VEGF) plays an important role in their pathogenesis. Currently, anti-VEGF antibodies are used as the standard treatment in the clinical treatment to prevent the neovascularization caused by VEGF with good clinical results.

Monoclonal antibodies (mAbs) have revolutionized the practice of biology and medicine. Since Professor Ferrara and others confirmed in 1989 that vascular endothelial growth factors can promote the proliferation of vascular endothelial cells, the VEGF family of cytokines, VEGF-A, B, C, D, E and PLGF, have been successively discovered. Among them, the binding of VEGF-A to its receptor triggers cascade reactions, promoting the division and proliferation of vascular endothelial cells and the formation of new blood vessels, and maintaining the survival of new blood vessels. VEGF-A is an inflammatory cell chemotactic factor that increases the vascular permeability. VEGF-A is detected to be highly expressed on surgically stripped wAMD neovascular membrane samples. As a major factor in CNV formation and progression, VEGF-A is a major therapeutic target for wAMD and DR. At present, anti-VEGF protein drugs for clinical intraocular injection have become the main drug for the treatment of ocular neovascularization. However, this treatment method requires long-term repeated injections, which brings a lot of pain and inconvenience to the patient.

Because the clinically recommended therapeutic dose require repeated injections, and fluctuating plasma concentrations make it difficult to maintain a stable plasma level. Due to the repeated injections, a patient needs to endure the tissue damage and pain caused by multiple injections. The cost of clinical treatment is also extremely high. This is a major drawback for clinical intraocular injection applications of protein drugs. Recombinant adeno-associated virus vector (rAAV)-guided gene therapy can overcome these shortcomings.

Adeno-associated virus (AAV) is a tiny (25 nanometer) replication-defective virus belonging to the family Parvovirus. It is a non-enveloped single-stranded linear DNA virus. Recombinant adeno-associated virus (rAAV) is constructed by replacing the wild-type viral genome with any gene or DNA sequence of interest. It can infect the cells in the replicative phase and the cells in the stationary phase and has the advantage of long-lasting transgene expression. No pathological and toxicological reactions caused by the virus can be observed. Therefore, rAAV is the most commonly used recombinant virus for gene therapy in recent decades of years.

### Summary of Invention

In an aspect, the present invention provides a recombinant nucleic acid encoding an anti-VEGF heavy chain. the coding sequence of the anti-VEGF heavy chain comprises a sequence having ≥90%, ≥95%, ≥96%, ≥97%, ≥98%, ≥99%, ≥99.5%, or 100% identity to one of the sequences as shown in SEQ ID NOs: 5-6 and 23-26. In some embodiments, the amino acid sequence of the anti-VEGF heavy chain comprises a sequence having ≥90%, ≥95%, ≥96%, ≥97%, ≥98%, ≥99%, ≥99.5% or 100% identity to one of the sequences as shown in SEQ ID NOs: 17 and 18. In some embodiments, the sequences of the complementarity determining region 1 (CDR1), the complementarity determining region 2 (CDR2), and the complementarity determining region 3 (CDR3) of the anti-VEGF heavy chain are identical to the sequences as shown in SEQ ID NO: 31, SEQ ID NO: 32, and SEQ ID NO: 33, respectively. In some embodiments, the anti-VEGF heavy chain does not include a constant region 2 (CH2) and a constant region 3 (CH3).

In an aspect, the present invention provides a recombinant nucleic acid encoding an anti-VEGF light chain. the coding sequence of the anti-VEGF light chain comprises a sequence having ≥90%, ≥95%, ≥96%, ≥97%, ≥98%, ≥99%, ≥99.5%, or 100% identity to one of the sequences as shown in SEQ ID NOs: 4 and 19 to 22. In some embodiments, the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO 16. In some embodiments, the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30 respectively.

In some embodiments, the recombinant nucleic acid further encodes an anti-VEGF light chain. the coding sequence of the anti-VEGF light chain comprises a sequence having ≥90%, ≥95%, ≥96%, ≥97%, ≥98%, ≥99%, ≥99.5%, or 100% identity to one of the sequences as shown in SEQ ID NOs: 4 and 19 to 22. In some embodiments, the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO 16. In some embodiments, the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30, respectively. In some embodiments, the combination of SEQ ID NOs: 4 and 19 to 22 with SEQ ID NOs: 5 and 6 and 23 to 26 is a combination selected from Table 1. In some embodiments, the combination of SEQ ID NOs: 4 and 19 to 22 with SEQ ID NOs: 5 and 6 and 23 to 26 is a combination selected from Table 3. In some embodiments, the coding sequence of the anti-VEGF heavy chain comprises a sequence having ≥90%, ≥95%, ≥96%, ≥97%, ≥98%, ≥99%, ≥99.5% or 100% identity to the sequence as shown in SEQ ID NO: 25, and the coding sequence of the anti-VEGF light chain comprises a sequence having ≥90%, ≥95%, ≥96, ≥97%, ≥98%, ≥99%, ≥99.5% or 100% identity to the sequence as shown in SEQ ID NO: 21. In some embodiments, the coding sequence of the anti-VEGF heavy chain comprises a sequence having ≥99%, ≥99.5% or 100% identity to the sequence as shown in SEQ ID NO: 25, and the anti-VEGF light chain coding sequence comprises a sequence having ≥99%, ≥99.5% or 100% identity to the sequence as shown in SEQ ID NO: 21. In some embodiments, the coding sequence of the anti-VEGF light chain is located upstream (at 5' end) of the coding sequence of the anti-VEGF heavy chain.

In some embodiments, the recombinant nucleic acid further comprises a coding sequence of a linker peptide between the coding sequence of the anti-VEGF light chain and the coding sequence of the anti-VEGF heavy chain. In some embodiments, the amino acid sequence of the linker peptide comprises any one of the sequences as shown in SEQ ID NOs: 14 and 15, or a sequence that differs from any one of the sequences as shown in SEQ ID NOs: 14 and 15 by no more than 1, no more than 2, or no more than 3 amino acids.

In some embodiments, the recombinant nucleic acid further comprises a promoter and/or an enhancer. The promoter and/or the enhancer is operably linked to an open reading frame encoding the anti-VEGF light chain and/or the anti-VEGF heavy chain. Optionally, the promoter is a chicken β-actin promoter; and optionally, the enhancer is a CMV enhancer. In some embodiments, the sequence of the promoter/enhancer comprises a sequence having ≥90%, ≥95%, ≥96%, ≥97%, ≥98%, ≥99%, ≥99.5% or 100% identity to the sequence as shown in SEQ ID NO: 1.

In some embodiment, the recombinant nucleic acid further comprises one or more signal peptide coding sequences. The signal peptide coding sequence is located upstream (at 5' end) of the coding sequence of the anti-VEGF heavy chain and/or the coding sequence of the anti-VEGF light chain. In some embodiments, the signal peptide coding sequence is connected to the 5' end of the coding sequence of the anti-VEGF heavy chain. Preferably, the signal peptide coding sequence connected to the anti-VEGF heavy chain comprises an amino acid sequence that is ≥90%, ≥95%, ≥96%, ≥97%, ≥98%, ≥99%, or 100% identical to any one of the sequences as shown in SEQ ID NOs: 8-13 and 34 and 35, and preferably ≥90%, ≥95%, ≥96%, ≥97%, ≥98%, ≥99%, or 100% identical to the sequence as shown in SEQ ID NO: 34. In some embodiments, the signal peptide coding sequence is connected to the 5' end of the anti-VEGF light chain. Preferably the signal peptide coding sequence connected to the anti-VEGF light chain comprises an amino acid sequence that is ≥90%, ≥95%, ≥96%, ≥97%, ≥98%, ≥99%, or 100% identical to any one of the sequence as shown in SEQ ID NOs: 8-13 and 34 and 35, and preferably ≥90%, ≥95%, ≥96%, ≥97%, ≥98%, ≥99%, or 100% identical to the sequence as shown in SEQ ID NO: 35.

In some embodiments, the recombinant nucleic acid further comprises an intron. Optionally, the intron is located between the sequence of the promoter/enhancer and the signal peptide coding sequence. In some embodiments, the sequence of the intron comprises a sequence that is ≥90%, ≥95%, ≥96%, ≥97%, ≥98%, ≥99%, ≥99.5% or 100% identical to any one of the sequence as shown in SEQ ID NOs: 2 and 3.

In some embodiments, the recombinant nucleic acid further comprises a poly(A) sequence. The poly(A) sequence is located downstream (at 3' end) of the coding sequence of the anti-VEGF heavy chain and/or the coding sequence of the anti-VEGF light chain; and optionally, the poly(A) sequence is a human growth hormone poly(A) sequence. In some embodiments, the poly(A) sequence comprises a sequence that is ≥90%, ≥95%, ≥96%, ≥97%, ≥98%, ≥99%, ≥99.5% or 100% identical to the sequence as shown in SEQ ID NO: 7.

In some embodiments, the recombinant nucleic acid further comprises a woodchuck hepatitis virus post-transcriptional regulatory element (WPRE) sequence, and the WPRE sequence is located between the open reading frame encoding the anti-VEGF light chain and/or the anti-VEGF heavy chain and the poly(A) sequence. In some embodiments, the WPRE sequence comprises a sequence that is ≥90%, ≥95%, ≥96%, ≥97%, ≥98%, ≥99%, ≥99.5% or 100% identical to the sequence as shown in SEQ ID NO: 27.

In an aspect, the present invention provides a viral vector. The viral vector comprises a recombinant nucleic acid as described herein. In some embodiments, the viral vector is an adeno-associated virus (AAV) vector. In some embodiments, the serotype of the adeno-associated virus vector is one or more selected from the group consisting of AAV1, AAV2, AAV3, AAV4, AAV5, AAV6, AAV7, AAV8, AAV9, AAVrh10, AAV2.7m8, AAV- DJ, AAV-PHP.B, AAV-PHP.S, and AAV-PHP.eB. In some embodiments, the viral vector further comprises an inverted terminal repeat (ITR) of AAV.

In an aspect, the present invention provides a viral virion. The viral virion comprises a recombinant nucleic acid as described herein. In some embodiments, the virion is an AAV virion. In some embodiments, the serotype of the AAV virion is one or more selected from the group consisting of AAV1, AAV2, AAV3, AAV4, AAV5, AAV6, AAV7, AAV8, AAV9, AAVrh10, AAV2.7m8, AAV-DJ, AAV-PHP.B, AAV-PHP.S, and AAV-PHP.eB.

In an aspect, the present invention provides a pharmaceutical preparation. The pharmaceutical preparation comprises the viral virion as described herein, the viral vector as described herein, or the recombinant nucleic acid as described herein, and a pharmaceutically acceptable carrier or excipient. In some embodiments, the pharmaceutical preparation is a liquid preparation.

In an aspect, the present invention provides use of the viral virion as described herein, the viral vector as described herein, or the recombinant nucleic acid as described herein in the preparation of medicines for treating eye diseases.

In an aspect, the present invention provides a method for treating an eye disease, which comprises administering to a subject in need thereof the pharmaceutical preparation, the viral virion, the viral vector, or the recombinant nucleic acid as described herein.

In some embodiments, the eye disease is choroidal neovascular disease. In some embodiments, the choroidal neovascular disease is age-related macular degeneration or diabetic retinal degeneration; and preferably, the diabetic retinal degeneration is diabetic macular edema. In some embodiments, the viral virion, the viral vector, the recombinant nucleic acid, or the pharmaceutical preparation is injected intraocularly, and preferably, intravitreally injected. In some embodiments, the viral virion, the viral vector, or the pharmaceutical preparation are capable of long-lasting expression of the anti-VEGF antibody or an antigen binding fragment thereof in the eye, to prevent the eye diseases caused by VEGF overexpression.

### Description of the Drawings

Fig. 1 shows the cell transfection, expression and screening of the constructed AAV plasmid vector, where 1 ug of ranibizumab (Lucentis) was used as a standard control.
Fig. 2a shows the results of the WB experiment that the anti-VEGF Fab expressed in vitro by the constructed AAV plasmid vector specifically binds to human VEGF, but does not bind to mouse serum.
Fig. 2b shows the results of the WB experiment that the anti-VEGF Fab expressed in vitro by the constructed AAV plasmid vector specifically binds to human VEGF, but does not bind to rat and mouse serum (in which the control group is a group incubated with Lucentis).
Fig. 3 shows the results of ELISA assay demonstrating that Lucentis specifically binds to human VEGF, but does not bind to rat and mouse serum.
Fig. 4 shows the results of gel electrophoresis of the AAVMDR viral vector produced by the constructed AAV plasmid vector produces through a three-plasmid system.
Fig. 5a shows the WB detection results of the anti-VEGF Fab expressed after the cells are infected with AAVMDR virus produced in this application. Fig. 5b shows the results of ELISA assay of the anti-VEGF Fab expressed after the cells are infected with AAVMDR virus produced in this application.
Fig. 6 shows the WB detection results of the anti-VEGF Fab expression in the vitreous cavity of mouse eyes by the AAVMDR virus produced in this application.
Fig. 7 shows the results of ELISA assay of the anti-VEGF Fab expressed after the vitreous cavity of mice are infected over gradient with AAVMDR virus produced in this application.
Fig. 8 shows the WB detection results of long-lasting anti-VEGF Fab expression in vivo by the AAVMDR virus described in this application.
Fig. 9 shows the fluorescence images of the whole-mount retina preparations derived from Rho-hVEGF transgenic mice 2 weeks and 4 weeks after infection with the AAVMDR virus described in this application, detecting the anti-angiogenesis effect of anti-VEGF Fab expressed by the AAVMDR virus in vivo.
Fig. 10 shows the cell transfection, expression and screening results after the codon optimization and construction onto the pcDNA3.1 plasmid described in this application.
Fig. 11 shows the cell transfection, expression and screening results after the codon optimization of the viral vector plasmid according to this application.
Fig. 12 shows the results of gel electrophoresis of the AAVOPT5-WPREm virus produced by the AAV plasmid vector according to this application through a three-plasmid system.
Fig. 13 shows the results of ELISA assay of the anti-VEGF Fab expressed after the cells are infected with AAVOPT and AAVMDR viruses produced in this application.
Fig. 14a shows the effect of the AAVOPT5-WPREm virus according to this application on the proliferation of HUVECs. Fig. 14b shows relevant statistical data.
Figs. 15a-15c shows the effect of the AAVOPT5-WPREm virus according to this application on the tube formation of HUVECs.
Fig. 16 shows the anti-vascular proliferation effect of anti-VEGF Fab expressed in mice after the Rho-hVEGF transgenic mice are infected with the AAVOPT5-WPREm virus described in this application.

### Detailed Description

In one aspect of the present invention, a high-expression rAAV expression vector is constructed, and a synthetic promoter is used to guide an anti-VEGF antibody gene fragment to be highly expressed in vivo. The expressed gene is packaged with AAV and delivered to and expressed in the vitreous cavity of the eye, for the treatment of ocular macular degeneration related to neovascularization. This route of administration of the gene drug, with a single administration, can guide the long-term expression of the therapeutic gene, and overcome the eyeball damage caused by repeated injections, so as to benefit the patients. Compared with current recombinant protein therapies, the medical expenses will be significantly reduced.

### Definitions

All technical and scientific terms used herein have the same meaning as understood by those skilled in the art to which the present invention belongs, unless otherwise defined. Although any methods and materials similar or equivalent to those described herein can be used in the practice or testing of the preparations and unit dosages herein, some methods and materials are now described. Unless otherwise stated, the techniques employed or considered herein are standard methods. The materials, methods, and examples are merely illustrative and not limiting.

As used herein and in the appended claims, the singular forms "alan" and "the" include plural objects, unless otherwise expressly indicated herein. Thus, for example, the reference to "a compound" includes a plurality of such agents, and the reference to "the salt" includes the reference to one or more salts (or a plurality of salts), their equivalents known to those skilled in the art, and the like.

As used herein, unless otherwise specified, the term "or" may be a conjunctive or a disjunctive conjunctive. As used herein, any embodiment may be combined with any other embodiment unless otherwise stated.

As used herein, certain inventive embodiments herein contemplate numerical ranges unless otherwise stated. When a range exists, the range includes the endpoints of the range. Additionally, each subrange and value within the range exists as if it is explicitly written out.

As used herein, the term "about" and its syntactic equivalents with respect to reference values and their syntactic equivalents may include a range of a value plus or minus 10% of the value, such as a range of the value plus or minus 10%, 9%, 8%, 7%, 6%, 5%, 4%, 3%, 2% or 1% of the value. For example, an amount of "about 10" includes an amount of 9 to 11.

The term "comprising" (and related terms such as "comprise", "comprises", "having" or "including") are not intended to exclude other embodiments, for example, the embodiments of any material composition, composition, method or process, or the like herein may be "composed of" or "substantially composed of" the features.

The term "subject" refers to mammals that have been or will be the subject of treatment, observation or experimentation. The term "mammal" is intended to have its standard meaning and to cover, for example, human beings, dogs, cats, sheep and cattle. The methods herein may be used in both human therapies and veterinary applications. In some embodiments, the subject is a human.

The term "treating" or "treatment" covers the administration of at least one compound disclosed herein or a pharmaceutically acceptable salt thereof to a mammalian subject (particularly a human subject) in need of such administration, and include (i) preventing the development of clinical symptoms of a disease such as cancer, (ii) alleviating the clinical symptoms of a disease such as cancer and/or (iii) preventive treatment for the prevention of a disease such as cancer attack.

The term "therapeutically effective amount" of a chemical entity herein refers to an amount effective to provide therapeutic benefits such as ameliorating the symptoms, slowing down the disease progression or preventing the disease when administered to a human subject or a non-human subject.

As used herein, the terms "nucleic acid" and "polynucleotide" may be used interchangeably unless otherwise specified.

The term "promoter" refers to a DNA sequence recognized by an enzyme/protein in a mammalian cell that is required to initiate the transcription of a particular gene. For example, a nucleotide sequence that binds to a RNA polymerase and/or any associated factors and initiates the transcription therein. Non-limiting examples of the promoter are described herein, such as, but not limited to, CB7 promoter, cytomegalovirus (CMV) promoter, Rous sarcoma virus (RSV) promoter, glial fibrillary acidic protein (GFAP) promoter, myelin basic protein (MBP) promoter, MMT promoter, EF-1α promoter, UB6 promoter, chicken β-actin promoter, CAG promoter, RPE65 promoter and opsin promoter, liver specific promoters such as thyroxine binding globulin (TBG) promoter, APOA2 promoter, SERPINA1 (hAAT) promoter, or mIR122 promoter, or muscle-specific promoters, such as human desmin promoter or Pitx3 promoter, and inducible promoters, such as a hypoxia-inducible promoter or a drug-inducible promoter. In some embodiments, the viral vector provided herein comprises one or more promoters that control transgene expression. In some embodiments, the promoter is a constitutive promoter. In some embodiments, the CB7 promoter includes other control elements that enhance transgene expression driven by the vector. In some embodiments, the promoter includes a TATA box. In some embodiments, the vector provided herein comprises one or more tissue-specific promoters (e.g., retinal pigment epithelium cell-specific promoter, CNS-specific promoter, liver-specific promoter, or muscle-specific promoter). In some embodiments, the viral vector provided herein comprises an RPE65 promoter or an opsin promoter (retinal cell/CNS specific promoter). In some embodiments, the promoter is an inducible promoter. In some embodiments, the promoter is a hypoxia-inducible promoter. In some embodiments, the promoter comprises a hypoxia-inducible factor (HIF) binding site. In some embodiments, the promoter comprises a HIF-1α binding site.

The term "enhancer" refers to a nucleotide sequence that increases the transcription level of a nucleic acid encoding a protein of interest (for example, an antibody specifically binding to VEGF or an antigen-binding antibody fragment thereof, or a soluble VEGF receptor). The enhancer sequence (50-1500 base pairs in length) typically increases the transcription level by providing an additional binding site for a transcription-related protein (e.g., transcription factor). In some embodiments, the enhancer sequence is found within an intronic sequence. Unlike a promoter sequence, the enhancer sequence can act at a greater distance from the transcription start site (e.g., compared with a promoter). Non-limiting examples of the enhancer include RSV enhancer, CMV enhancer, or SV40 enhancer.

The term "intron" refers to a fragment or an interpolated sequence in a gene that has no coding performance. For example, chicken β-actin intron, minute virus of mouse (MVM) intron, human factor IX intron, β-globin cleavage donor/immunoglobulin heavy chain cleavage acceptor intron, adenovirus cleavage donor/immunoglobulin cleavage acceptor intron, SV40 late cleavage donor/cleavage acceptor (19S/16S) intron, hybrid adenovirus cleavage donor/IgG cleavage receptor intron, and polyadenylation signals, such as rabbit β-globin polyadenylation signal, human growth hormone (hGH) polyadenylation signal, SV40 late polyadenylation signal, synthetic polyadenylation (SPA) signal and bovine growth hormone (bGH) polyadenylation signal, etc. In some embodiments, the intron is a synthetic intron 1 (SEQ ID NO: 2). In some embodiments, the intron is a synthetic intron 1 (SEQ ID NO: 3).

The term "signal peptide" refers to a sequence that exists at the N-terminus of a newly secreted protein but not in a naturally occurring mature protein. After the signal peptide is translated, the "signal peptide" is lysed by a protease (e.g., a signal peptidase). One approach is to use a signal peptide from a protein that is homologous to the protein expressed. For example, a human antibody signal peptide can be used to express IgG in CHO or other cells. Another approach is to identify a signal peptide optimized for a particular host cell used for expression. The signal peptides can be interchanged between different proteins or even between proteins of different organisms, and generally a signal sequence of the most abundantly secreted protein of a cell type is used for protein expression. For example, a signal peptide for human albumin, the most abundant protein in plasma, is found to significantly increase the protein production yield in CHO cells. In a preferred embodiment, the signal sequence is fused to both the heavy and light chain sequences. Preferred sequences are signal peptides for expression in ocular/CNS tissues. Non-limiting examples of the signal peptide include: signal peptide 1 (SEQ ID NO: 8), signal peptide 2 (SEQ ID NO: 9), signal peptide 3 (SEQ ID NO: 10), signal peptide 4 (SEQ ID NO: 11), signal peptide 5 (SEQ ID NO: 12), signal peptide 6 (SEQ ID NO: 13), signal peptide 7 (SEQ ID NO: 34), and signal peptide 8 (SEQ ID NO: 35).

The term "linker peptide" refers to a coding sequence that separates the heavy and light chains. For example, the furin-F2A linker peptide (Fang et al., 2005, Nature Biotechnology 23: 584-590, and Fang, 2007, Mol Ther 15:1153-9, each of which is hereby incorporated by reference in its entirety) can be bound into an expression cassette to separate the heavy and light chain coding sequences. Additional linker peptides that may be used include, but are not limited to, linker peptide 1 (SEQ ID NO: 14) or linker peptide 2 (SEQ ID NO: 15).

The term "woodchuck hepatitis virus post-transcriptional regulatory element (WPRE)" is a DNA sequence and a tripartite regulatory element with gamma, alpha, and beta components that, when transcribed, creates a tertiary structure enhancing expression. This sequence is often used to increase the expression of a gene delivered by a viral vector. When added to the 3' untranslated region (UTR) of a mammalian expression cassette, it can significantly enhance the mRNA stability and protein yield. A WPRE sequence that can be used includes, but is not limited to, SEQ ID NO: 27.

The term "poly(A) sequence" or "polyadenylation signal sequence" refers to a sequence that triggers the cleavage of mRNA by an endonuclease and the addition of a series of adenosines to the 3' end of the cleaved mRNA. In some embodiments, the poly(A) signal sequence is located at the 3' terminus of a nucleic acid sequence encoding an antibody heavy chain, an antibody light chain, or an antigen-binding antibody fragment. The poly(A) tail and the protein bound thereto protect the mRNA from degradation by an exonuclease. Polyadenylation is also important for transcription termination, export of mRNA from the nucleus, and translation. After DNA is transcribed into RNA, polyadenylation occurs in the nucleus, but can also occur later in the cytoplasm. After the transcription is terminated, the mRNA strand is cleaved by an endonuclease complex associated with a RNA polymerase. There are several poly(A) signal sequences that can be used, including those derived from the group consisting of: bovine growth hormone (bgh), mouse beta-globin, mouse alpha-globin, human collagen, polyomavirus, herpes simplex virus thymidine kinase gene (HSV TK), IgG heavy chain gene polyadenylation signal, human growth hormone (hGH), and SV40 poly(A) sites, such as SV40 late and early poly(A) sites, etc. In some embodiments, the poly(A) sequence is human growth hormone polyadenosine hGH Poly(A) sequence (SEQ ID NO: 7).

### Recombinant nucleic acid

In one aspect, the present invention is useful in the expression of an anti-VEGF antibody or an antigen-binding fragment thereof. In some embodiments, the antibody or antigen-binding fragment thereof herein is based on the amino acid sequence of ranibizumab published on Drug Bank (DB01270). In some embodiments, the antibody or antigen-binding fragment thereof herein lacks one or more of the antibody constant regions (CHs). For example, the sequence of ranibizumab lacks a constant region 2 (CH2) and a constant region 3 (CH3). Thus, in some embodiments, the antibody herein does not comprise the constant region 2 (CH2). Thus, in some embodiments, the antibody herein does not comprise the constant region 3 (CH3). In some embodiments, the antibody herein does not comprise both the constant region 2 (CH2) and the constant region 3 (CH3). In some embodiments, the antibody herein comprises a heavy chain variable region (VH), a heavy chain constant region 1 (CH1), a light chain variable region (VL), and a light chain constant region (CL), but does not include the constant region 2 (CH2) and the constant region 3 (CH3).

In some embodiments, the antibody or antigen-binding fragment thereof herein is selected from the group consisting of a polyclonal antibody, a monoclonal antibody, a humanized antibody, a human antibody, a single chain Fv (scFv) antibody, a single domain antibody, Fab, F(ab') 2, a single-chain diabody, an antibody mimic and an antibody variable domain. In some embodiments, the antibody herein is Fab.

In one aspect, the present invention provides a recombinant nucleic acid. In some embodiments, the recombinant nucleic acid comprises an antibody gene fragment expression cassette. In some embodiments, the recombinant nucleic acid includes one or more of the following elements: a synthetic promoter; an anti-VEGF antibody gene fragment guided by a signal peptide; a self-cleaving linker peptide with a human endonuclease added to the 5-end; and a human growth hormone polyadenosine (poly(A)) tail signal. In some embodiments, both ends of the expression cassette are packaged by an AAV2-type inverted terminal repeat.

In some embodiments, the recombinant nucleic acid herein comprises a coding sequence of an anti-VEGF heavy chain. In some embodiments, the anti-VEGF heavy chain does not include a constant region 2 (CH2). In some embodiments, the anti-VEGF heavy chain does not include a constant region 3 (CH3). In some embodiments, the anti-VEGF heavy chain comprises
a) a sequence having ≥90%, ≥95%, ≥96%, ≥97%, ≥98%, ≥99%, ≥99.5% or 100% identity to the sequence as shown in SEQ ID NO: 5, preferably, a sequence having ≥99% identity to the sequence as shown in SEQ ID NO: 5, more preferably, a sequence having ≥99.5% identity to the sequence as shown in SEQ ID NO: 5, and more preferably, a sequence having 100% identity to the sequence as shown in SEQ ID NO: 5;
b) a sequence having ≥90%, ≥95%, ≥96%, ≥97%, ≥98%, ≥99%, ≥99.5%or 100% identity to the sequence as shown in SEQ ID NO: 6, preferably, a sequence having ≥99% identity to the sequence as shown in SEQ ID NO: 6, more preferably, a sequence having ≥99.5% identity to the sequence as shown in SEQ ID NO: 6, and more preferably, a sequence having 100% identity to the sequence as shown in SEQ ID NO: 6;
c) a sequence having ≥90%, ≥95%, ≥96%, ≥97%, ≥98%, ≥99%, ≥99.5%or 100% identity to the sequence as shown in SEQ ID NO: 23, preferably, a sequence having ≥99% identity to the sequence as shown in SEQ ID NO: 23, more preferably, a sequence having ≥99.5% identity to the sequence as shown in SEQ ID NO: 23, and more preferably, a sequence having 100% identity to the sequence as shown in SEQ ID NO: 23;
d) a sequence having ≥90%, ≥95%, ≥96%, ≥97%, ≥98%, ≥99%, ≥99.5%or 100% identity to the sequence as shown in SEQ ID NO: 24, preferably, a sequence having ≥99% identity to the sequence as shown in SEQ ID NO: 24, more preferably, a sequence having ≥99.5% identity to the sequence as shown in SEQ ID NO: 24, and more preferably, a sequence having 100% identity to the sequence as shown in SEQ ID NO: 24;
e) a sequence having ≥90%, ≥95%, ≥96%, ≥97%, ≥98%, ≥99%, ≥99.5%or 100% identity to the sequence as shown in SEQ ID NO: 25, preferably, a sequence having ≥99% identity to the sequence as shown in SEQ ID NO: 25, more preferably, a sequence having ≥99.5% identity to the sequence as shown in SEQ ID NO: 25, and more preferably, a sequence having 100% identity to the sequence as shown in SEQ ID NO: 25; or
f) a sequence having ≥90%, ≥95%, ≥96%, ≥97%, ≥98%, ≥99%, ≥99.5%or 100% identity to the sequence as shown in SEQ ID NO: 26, preferably, a sequence having ≥99% identity to the sequence as shown in SEQ ID NO: 26, more preferably, a sequence having ≥99.5% identity to the sequence as shown in SEQ ID NO: 26, and more preferably, a sequence having 100% identity to the sequence as shown in SEQ ID NO: 26.

In some embodiments, the recombinant nucleic acid herein comprises a coding sequence of an anti-VEGF light chain. In some embodiments, the anti-VEGF light chain comprises:
a) a sequence having ≥90%, ≥95%, ≥96%, ≥97%, ≥98%, ≥99%, ≥99.5%or 100% identity to the sequence as shown in SEQ ID NO: 4, preferably, a sequence having ≥99% identity to the sequence as shown in SEQ ID NO: 4, more preferably, a sequence having ≥99.5% identity to the sequence as shown in SEQ ID NO: 4, and more preferably, a sequence having 100% identity to the sequence as shown in SEQ ID NO: 4;
b) a sequence having ≥90%, ≥95%, ≥96%, ≥97%, ≥98%, ≥99%, ≥99.5%or 100% identity to the sequence as shown in SEQ ID NO: 19, preferably, a sequence having ≥99% identity to the sequence as shown in SEQ ID NO: 19, more preferably, a sequence having ≥99.5% identity to the sequence as shown in SEQ ID NO: 19, and more preferably, a sequence having 100% identity to the sequence as shown in SEQ ID NO: 19;
c) a sequence having ≥90%, ≥95%, ≥96%, ≥97%, ≥98%, ≥99%, ≥99.5%or 100% identity to the sequence as shown in SEQ ID NO: 20, preferably, a sequence having ≥99% identity to the sequence as shown in SEQ ID NO: 20, more preferably, a sequence having ≥99.5% identity to the sequence as shown in SEQ ID NO: 20, and more preferably, a sequence having 100% identity to the sequence as shown in SEQ ID NO: 20;
d) a sequence having ≥90%, ≥95%, ≥96%, ≥97%, ≥98%, ≥99%, ≥99.5%or 100% identity to the sequence as shown in SEQ ID NO: 21, preferably, a sequence having ≥99% identity to the sequence as shown in SEQ ID NO: 21, more preferably, a sequence having ≥99.5% identity to the sequence as shown in SEQ ID NO: 21, and more preferably, a sequence having 100% identity to the sequence as shown in SEQ ID NO: 21; or
e) a sequence having ≥90%, ≥95%, ≥96%, ≥97%, ≥98%, ≥99%, ≥99.5%or 100% identity to the sequence as shown in SEQ ID NO: 22, preferably, a sequence having ≥99% identity to the sequence as shown in SEQ ID NO: 22, more preferably, a sequence having ≥99.5% identity to the sequence as shown in SEQ ID NO: 22, and more preferably, a sequence having 100% identity to the sequence as shown in SEQ ID NO: 22.

In some embodiments, the recombinant nucleic acids herein comprise a combination of coding sequences of anti-VEGF heavy chain and anti-VEGF light chain selected from Table 1:

**Table 1: Combination of coding sequences of anti-VEGF heavy chain and anti-VEGF light chain**

| | | | | | | |
|---|---|---|---|---|---|---|
| | The coding sequence of the anti-VEGF light chain comprises a sequence having ≥90%, ≥95%, ≥96%, ≥97%, ≥98%, ≥99%, ≥99.5% or 100% identity to the corresponding sequence as shown in SEQ ID NO,, preferably a sequence having ≥99% identity to the corresponding sequence as shown in SEQ ID NO, further preferably a sequence having ≥99.5% identity to the corresponding sequence as shown in SEQ ID NO, and further preferably a sequence having 100% identity to the corresponding sequence as shown in SEQ ID NO. | | | | | |
| The coding sequence of the anti-VEGF heavy chain comprises a sequence having ≥90%, ≥95%, ≥96%, ≥97%, ≥98%, ≥99%, ≥99.5% or 100% identity to the corresponding sequence as shown in SEQ ID NO, preferably a sequence having ≥99% identity to the corresponding sequence as shown in SEQ ID NO, further preferably a sequence having ≥99.5% identity to the corresponding sequence as shown in SEQ ID NO, and further preferably a sequence having 100% identity to the corresponding sequence as shown in SEQ ID NO. | | SEQ ID NO: 4 | SEQ ID NO: 19 | SEQ ID NO: 20 | SEQ ID NO: 21 | SEQ ID NO: 22 |
| | SEQ ID NO: 5 | X | X | X | X | X |
| | SEQ ID NO: 6 | X | X | X | X | X |
| | SEQ ID NO: 23 | X | X | X | X | X |
| | SEQ ID NO: 24 | X | X | X | X | X |
| | SEQ ID NO: 25 | X | X | X | X | X |
| | SEQ ID NO: 26 | X | X | X | X | X |

| | | | | | | |
|---|---|---|---|---|---|---|
| "X" indicates an optional combination of coding sequences of anti-VEGF heavy chain and anti-VEGF light chain encoding sequence. | | | | | | |

In some embodiments, the recombinant nucleic acid herein comprises a coding sequence for an anti-VEGF heavy chain and a coding sequence for an anti-VEGF light chain. the coding sequence of the anti-VEGF heavy chain comprises a sequence having ≥90%, ≥95%, ≥96%, ≥97%, ≥98%, ≥99%, ≥99.5% or 100% identity to the sequence as shown in SEQ ID NO: 25, and the coding sequence of the anti-VEGF light chain comprises a sequence having ≥90%, ≥95%, ≥96, ≥97%, ≥98%, ≥99%, ≥99.5% or 100% identity to the sequence as shown in SEQ ID NO: 21. In some embodiments, the coding sequence of the anti-VEGF heavy chain comprises a sequence having ≥ 95% identity to the sequence as shown in SEQ ID NO: 25, and the coding sequence of the anti-VEGF light chain comprises a sequence having ≥ 95% identity to the sequence as shown in SEQ ID NO: 21. In some embodiments, the coding sequence of the anti-VEGF heavy chain comprises a sequence having ≥99% identity to the sequence as shown in SEQ ID NO: 25, and the coding sequence of the anti-VEGF light chain comprises a sequence having ≥ 95% identity to the sequence as shown in SEQ ID NO: 21. In some embodiments, the coding sequence of the anti-VEGF heavy chain comprises a sequence having ≥95% identity to the sequence as shown in SEQ ID NO: 25, and the coding sequence of the anti-VEGF light chain comprises a sequence having ≥ 99% identity to the sequence as shown in SEQ ID NO: 21. In some embodiments, the coding sequence of the anti-VEGF heavy chain comprises a sequence having ≥99% identity to the sequence as shown in SEQ ID NO: 25, and the coding sequence of the anti-VEGF light chain comprises a sequence having ≥ 99% identity to the sequence as shown in SEQ ID NO: 21. In some embodiments, the coding sequence of the anti-VEGF heavy chain comprises a sequence having ≥99.5% identity to the sequence as shown in SEQ ID NO: 25, and the coding sequence of the anti-VEGF light chain comprises a sequence having ≥ 99.5% identity to the sequence as shown in SEQ ID NO: 21. In some embodiments, the coding sequence of the anti-VEGF heavy chain comprises a sequence having or 100% identity to the sequence as shown in SEQ ID NO: 25, and the coding sequence of the anti-VEGF light chain comprises a sequence having ≥99% identity to the sequence as shown in SEQ ID NO: 21. In some embodiments, the coding sequence of the anti-VEGF heavy chain comprises a sequence having ≥99% identity to the sequence as shown in SEQ ID NO: 25, and the coding sequence of the anti-VEGF light chain comprises a sequence having 100% identity to the sequence as shown in SEQ ID NO: 21. In some embodiments, the coding sequence of the anti-VEGF heavy chain comprises a sequence having ≥99.5% identity to the sequence as shown in SEQ ID NO: 25, and the coding sequence of the anti-VEGF light chain comprises a sequence having 100% identity to the sequence as shown in SEQ ID NO: 21. In some embodiments, the coding sequence of the anti-VEGF heavy chain comprises a sequence having or 100% identity to the sequence as shown in SEQ ID NO: 25, and the coding sequence of the anti-VEGF light chain comprises a sequence having ≥99.5% identity to the sequence as shown in SEQ ID NO: 21. In some embodiments, the coding sequence of the anti-VEGF heavy chain comprises a sequence having 100% identity to the sequence as shown in SEQ ID NO: 25, and the coding sequence of the anti-VEGF light chain comprises a sequence having 100% identity to the sequence as shown in SEQ ID NO: 21.

In some embodiments, the amino acid sequence of the anti-VEGF heavy chain comprises a sequence having ≥90%, ≥95%, ≥96%, ≥97%, ≥98%, ≥99%, ≥99.5% or 100% identity to the sequence as shown in SEQ ID: 17, preferably a sequence having ≥99% identity to the sequence as shown in SEQ ID: 17, further preferably a sequence having ≥99.5% identity to the sequence as shown in SEQ ID: 17, and further preferably a sequence having 100% identity to the sequence as shown in SEQ ID: 17. In some embodiments, the amino acid sequence of the anti-VEGF heavy chain comprises a sequence having ≥90%, ≥95%, ≥96%, ≥97%, ≥98%, ≥99%, ≥99.5% or 100% identity to the sequence as shown in SEQ ID NO: 18, preferably, a sequence having ≥99% identity to the sequence as shown in SEQ ID NO: 18, more preferably, a sequence having ≥99.5% identity to the sequence as shown in SEQ ID NO: 17, and more preferably, a sequence having 100% identity to the sequence as shown in SEQ ID NO: 18.

In some embodiments, the sequences of the complementarity determining region 1 (CDR1), the complementarity determining region 2 (CDR2), and the complementarity determining region 3 (CDR3) of the anti-VEGF heavy chain are identical to the CDR1, CDR2 and CDR3 sequences shown in SEQ ID NO: 17. In some embodiments, the anti-VEGF heavy chain includes the following three complementarity determining regions (CDRs):
CDR1: GYDFTHYGMN (SEQ ID NO: 31)
CDR2: WINTYTGEPTYAADFKR (SEQ ID NO: 32)
CDR3: YPYYYGTSHWYFDV (SEQ ID NO: 33)

In some embodiments, the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID: 16, preferably a sequence having ≥99% identity to the sequence as shown in SEQ ID: 16, further preferably a sequence having ≥99.5% identity to the sequence as shown in SEQ ID: 16, and further preferably a sequence having 100% identity to the sequence as shown in SEQ ID: 16. In some embodiments, the sequences of the complementarity determining region 1 (CDR1), the complementarity determining region 2 (CDR2), and the complementarity determining region 3 (CDR3) of the anti-VEGF light chain are identical to the CDR1, CDR2 and CDR3 sequences shown in SEQ ID NO: 16. In some embodiments, the anti-VEGF light chain includes the following three complementarity determining regions (CDRs):
CDR1: SASQDISNYLN (SEQ ID NO: 28)
CDR2: FTSSLHS (SEQ ID NO: 29)
CDR3: QQYSTVPWT (SEQ ID NO: 30)

In some embodiments, the recombinant nucleic acid herein includes a coding sequence for an anti-VEGF heavy chain and a coding sequence for an anti-VEGF light chain. The coding sequence for the anti-VEGF light chain is located upstream (at 5' end) of the coding sequence for the anti-VEGF heavy chain. In some embodiments, the coding sequence of the anti-VEGF light chain is located downstream (at 3' end) of the coding sequence of the anti-VEGF heavy chain.

In some embodiments, the recombinant nucleic acid further comprises a coding sequence of a linker peptide between the coding sequence of the anti-VEGF light chain and the coding sequence of the anti-VEGF heavy chain. In some embodiments, the linker peptide is a self-cleavable linker peptide. In some embodiments, the linker peptide is a 2A self-cleavable linker peptide, such as T2A, P2A, E2A, and F2A. In some embodiments, the amino acid sequence of the linker peptide comprises a sequence having ≥90%, ≥95%, ≥96%, ≥97%, ≥98%, ≥99%, or 100% identity to the sequence as shown in SEQ ID NO 14. In some embodiments, the amino acid sequence of the linker peptide comprises a sequence having ≥90%, ≥95%, ≥96%, ≥97%, ≥98%, ≥99%, or 100% identity to the sequence as shown in SEQ ID NO: 15.

In some embodiments, the recombinant nucleic acid further comprises a promoter operably linked to an open reading frame (ORF) encoding the anti-VEGF light chain and/or the anti-VEGF heavy chain. In some embodiments, the promoter is the chicken β-actin promoter.

In some embodiments, the recombinant nucleic acid further comprises an enhancer operably linked to an open reading frame (ORF) encoding the anti-VEGF light chain and/or the anti-VEGF heavy chain. In some embodiments, the enhancer is a CMV enhancer.

In some embodiments, the recombinant nucleic acid further comprises a promoter/enhancer sequence operably linked to an open reading frame, where the promoter/enhancer sequence comprises a sequence having ≥90%, ≥95%, ≥96%, ≥97%, ≥98%, ≥99%, ≥99.5% or 100% identity to the sequence as shown in SEQ ID NO: 1.

In some embodiments, the recombinant nucleic acid comprises an intron. In some embodiments, the intron is located between the sequence of the promoter/enhancer and the coding sequence of the (first) signal peptide. In some embodiments, the sequence of the intron comprises a sequence having ≥90%, ≥95%, ≥96%, ≥97%, ≥98%, ≥99%, ≥99.5% or 100% identity to the sequence as shown in SEQ ID NO 2. In some embodiments, the sequence of the intron comprises a sequence that is ≥90%, ≥95%, ≥96%, ≥97%, ≥98%, ≥99%, ≥99.5% or 100% identical to the sequence as shown in SEQ ID NO: 3.

In some embodiments, the open reading frame encoding the anti-VEGF light chain and/or the anti-VEGF heavy chain in the recombinant nucleic acid further comprises one or more signal peptide coding sequences. In some embodiments, the (first) signal peptide coding sequence is located at the 5' end of the open reading frame. In some embodiments, the signal peptide coding sequence is located upstream (at 5' end) of the coding sequence of the anti-VEGF heavy chain and/or the coding sequence of the anti-VEGF light chain.

In some embodiments, the signal peptide comprises an amino acid sequence that is ≥90%, ≥95%, ≥96%, ≥97%, ≥98%, ≥99%, or 100% identical to any one of the sequence as shown in SEQ ID NOs: 8 to 13 and 34 and 35. In some embodiments, the signal peptide comprises an amino acid sequence that is the same as the sequence as shown in SEQ ID NO: 8, or differs from sequence as shown in SEQ ID NO: 8 by no more than 1, no more than 2, no more than 3, or no more than 4 amino acids. In some embodiments, the signal peptide comprises an amino acid sequence that is the same as the sequence as shown in SEQ ID NO: 9, or differs from sequence as shown in SEQ ID NO: 9 by no more than 1, no more than 2, no more than 3, or no more than 4 amino acids. In some embodiments, the signal peptide comprises an amino acid sequence that is the same as the sequence as shown in SEQ ID NO: 10, or differs from sequence as shown in SEQ ID NO: 10 by no more than 1, no more than 2, no more than 3, or no more than 4 amino acids. In some embodiments, the signal peptide comprises an amino acid sequence that is the same as the sequence as shown in SEQ ID NO: 11, or differs from sequence as shown in SEQ ID NO: 11 by no more than 1, no more than 2, no more than 3, or no more than 4 amino acids. In some embodiments, the signal peptide comprises an amino acid sequence that is the same as the sequence as shown in SEQ ID NO: 12, or differs from sequence as shown in SEQ ID NO: 12 by no more than 1, no more than 2, no more than 3, or no more than 4 amino acids. In some embodiments, the signal peptide comprises an amino acid sequence that is the same as the sequence as shown in SEQ ID NO: 13, or differs from sequence as shown in SEQ ID NO: 13 by no more than 1, no more than 2, no more than 3, or no more than 4 amino acids. In some embodiments, the signal peptide comprises an amino acid sequence that is the same as the sequence as shown in SEQ ID NO: 34, or differs from sequence as shown in SEQ ID NO: 34 by no more than 1, no more than 2, no more than 3, or no more than 4 amino acids. In some embodiments, the signal peptide comprises an amino acid sequence that is the same as the sequence as shown in SEQ ID NO: 35, or differs from sequence as shown in SEQ ID NO: 35 by no more than 1, no more than 2, no more than 3, or no more than 4 amino acids.

In some embodiments, the recombinant nucleic acid further comprises a poly(A) sequence. In some embodiments, the poly(A) sequence is located downstream (at 3' end) of the coding sequence of the anti-VEGF heavy chain and/or the coding sequence of the anti-VEGF light chain. In some embodiments, the poly(A) sequence is operably linked to the coding sequence of the anti-VEGF heavy chain and/or the coding sequence of the anti-VEGF light chain. In some embodiments, the poly(A) sequence is human growth hormone polyadenosine. In some embodiments, the poly(A) sequence comprises a sequence that is ≥90%, ≥95%, ≥96%, ≥97%, ≥98%, ≥99%, ≥99.5% or 100% identical to the sequence as shown in SEQ ID NO: 7.

In some embodiments, the recombinant nucleic acid further comprises a woodchuck hepatitis virus post-transcriptional regulatory element (WPRE) sequence. In some embodiments, the WPRE sequence is operably linked to the coding sequence of the anti-VEGF heavy chain and/or the coding sequence of the anti-VEGF light chain. In some embodiments, the WPRE sequence comprises a sequence that is ≥90%, ≥95%, ≥96%, ≥97%, ≥98%, ≥99%, ≥99.5% or 100% identical to the sequence as shown in SEQ ID NO: 27.

In some embodiments, the recombinant nucleic acids herein comprise 1) one or more nucleotide sequences selected from Table 2 below; and/or 2) one or more sequences encoding the amino acid sequences selected from Table 2 below.

**Table 2: DNA/amino acid sequences included herein**

| **Serial number** | **Sequence name** | **DNA/amino acid sequence** |
|---|---|---|
| SEQ ID NO: 1 | CMV enhancer and chicken β-actin promoter | DNA sequence |
| SEQ ID NO: 2 | Synthetic intron 1 | DNA sequence |
| SEQ ID NO: 3 | Synthetic intron 2 | DNA sequence |
| SEQ ID NO: 7 | Human growth hormone polyadenosine | DNA sequence |
| SEQ ID NO: 8: | Signal peptide 1 | Amino acid sequence |
| SEQ ID NO: 9: | Signal peptide 2 | Amino acid sequence |
| SEQ ID NO: 10: | Signal peptide 3 | Amino acid sequence |
| SEQ ID NO: 11: | Signal peptide 4 | Amino acid sequence |
| SEQ ID NO: 12 | Signal peptide 5 | Amino acid sequence |
| SEQ ID NO: 13 | Signal peptide 6 | Amino acid sequence |
| SEQ ID NO: 34 | Signal peptide 7 | Amino acid sequence |
| SEQ ID NO: 35 | Signal peptide 8 | Amino acid sequence |
| SEQ ID NO: 14 | Linker peptide 1 | Amino acid sequence |
| SEQ ID NO: 15 | Linker peptide 2 | Amino acid sequence |
| SEQ ID NO: 27 | Woodchuck hepatitis virus post-transcriptional regulatory element (WPRE) | DNA sequence |
| SEQ ID NO: 4 | Anti-VEGF light chain 1 | DNA sequence |
| SEQ ID NO: 19 | Anti-VEGF light chain 2 | DNA sequence |
| SEQ ID NO: 20 | Anti-VEGF light chain 3 | DNA sequence |
| SEQ ID NO: 21 | Anti-VEGF light chain 4 | DNA sequence |
| SEQ ID NO: 22 | Anti-VEGF light chain 5 | DNA sequence |
| SEQ ID NO: 5 | Anti-VEGF heavy chain 1 | DNA sequence |
| SEQ ID NO: 6 | Anti-VEGF heavy chain 2 | DNA sequence |
| SEQ ID NO: 23 | Anti-VEGF heavy chain 3 | DNA sequence |
| SEQ ID NO: 24 | Anti-VEGF heavy chain 4 | DNA sequence |
| SEQ ID NO: 25 | Anti-VEGF heavy chain 5 | DNA sequence |
| SEQ ID NO: 26 | Anti-VEGF heavy chain 6 | DNA sequence |
| SEQ ID NO: 16 | Anti-VEGF light chain | Amino acid sequence |
| SEQ ID NO: 17 | Anti-VEGF heavy chain a | Amino acid sequence |
| SEQ ID NO: 18 | Anti-VEGF heavy chain b | Amino acid sequence |

In some embodiments, the recombinant nucleic acid includes, from the 5' to 3' direction, the enhancer and promoter sequences, the intron sequence, the coding sequence of the first signal peptide, the coding sequence of the anti-VEGF light chain, the coding sequence of the linker peptide, the coding sequence of the second signal peptide, the coding sequence of the anti-VEGF heavy chain, and the poly(A) signal sequence. In some embodiments, the recombinant nucleic acid further includes a WPRE sequence preceding the poly(A) signal sequence. In some embodiments, the WPRE sequence follows an open reading frame encoding the anti-VEGF light/heavy chain. In some embodiments, no other nucleotide sequences exist between two adjacent sequences of the above nucleotide sequences.

### Nucleic acid

The nucleic acid and polypeptide herein are useful in the method and vector of the present disclosure. In some embodiments, an aptamer that specifically binds to the nucleic acid and polypeptide herein can be used in the method and vector of the present disclosure. As used herein, the nucleic acid may comprise deoxyribonucleotide (DNA) or ribonucleotide (RNA) (whether in monomeric or polymeric form, naturally occurring or non-naturally occurring, double-stranded or single-stranded, coding (e.g. translated gene) or non-coding (e.g. regulatory region)), or any fragment, derivative, mimetic or complement thereof. In some embodiments, the nucleic acid can include oligonucleotides, nucleotides, polynucleotides, nucleic acid sequences, genomic sequences, complementary DNA (cDNA), antisense nucleic acids, DNA regions, probes, primers, genes, regulatory regions, introns, exons, open reading frames, binding sites, target nucleic acids and allele-specific nucleic acids. As used herein, "heterozygote" includes double chains formed between any of the aforementioned nucleic acids (within the same type or across different types), including DNA-DNA, DNA-RNA, and RNA-RNA, etc.

As used herein, the "isolated" nucleic acid is isolated from the nucleic acid of a commonly flanked gene or nucleotide sequence (e.g., in a genomic sequence) and/or has been fully or partially purified from other transcription sequences (e.g., in RNA banks). For example, an isolated nucleic acid in the present disclosure may be substantially isolated from the complex cellular environment in which it naturally occurs, or the culture medium when produced by recombinant techniques, or the chemical precursors or other chemicals when chemically synthesized. In some cases, the isolated material may form part of a composition, for example, a crude extract, a buffer system, or a reagent mixture containing other substances. In some embodiments, the material can be purified to be substantially pure by methods known in the art, such as, polyacrylamide gel electrophoresis (PAGE) or column chromatography (e.g., HPLC). With regard to the genomic DNA (gDNA), the term "isolated" may also refer to the separation of a nucleic acid from a chromosome that is naturally associated with the genomic DNA. For example, an isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of nucleotides flanking a nucleic acid molecule in the gDNA of the cell from which the nucleic acid molecule is derived.

The nucleic acid may be fused to other coding or regulatory sequences and may be considered isolated. For example, recombinant DNA contained in a vector is included in the definition of "isolated" as used herein. In some embodiments, an isolated nucleic acid can include a recombinant DNA molecule in a heterologous host cell or heterologous organism, as well as a partially or substantially purified DNA molecule in a solution. The isolated nucleic acid also encompasses an in-vivo and an in-vitro RNA transcript of the DNA molecule of the present disclosure. The isolated nucleic acid molecule or nucleotide sequence can be synthesized chemically or by recombinant means. Such an isolated nucleotide sequence can be used, for example, to produce an encoded polypeptide which is used as a probe for isolating a homologous sequence (e.g., from other mammalian species), for gene mapping (e.g., by in-situ hybridization with a chromosome), or for detecting the gene expression in a tissue (e.g., human tissue), for example, by Northern blot analysis or other hybridization techniques disclosed herein. The present disclosure also relates to a nucleic acid sequence that hybridizes with the nucleotide sequence herein under highly stringent hybridization conditions (such as for selective hybridization). Such a nucleic acid sequence can be detected and/or isolated by allele- or sequence-specific hybridization (e.g., under highly stringent conditions). Stringent conditions and methods for nucleic acid hybridization are well known to those skilled in the art (see, e.g., Current Protocols in Molecular Biology, Ausubel, F. et al., John Wiley & Sons, (1998), and Kraus, M. and Aaronson, S., Methods Enzymol., 200:546-556 (1991)), the entire teachings of which are incorporated herein by reference.

The calculation of "identity" or "percent identity" between two or more nucleotide or amino acid sequences may be determined by aligning the sequences for optimal comparison purposes (e.g., a gap may be introduced in the sequence of the first sequence). The nucleotides at corresponding positions are then compared, and the percent identity between the two sequences is a function of the number of identical positions shared by the sequences (i.e. % identity = number of identical positions / total number of positions x 100. For example, if a position in a first sequence and a corresponding position in a second sequence are occupied by the same nucleotide, then the molecules at that position are identical. The percent identity between two sequences is a function of the number of identical positions shared by the sequences, taking into account the number of gaps and the length of each gap that need to be introduced to achieve an optimal alignment of the two sequences.

In some embodiments, the length of the sequences aligned for comparison purposes is at least 30%, at least 40%, at least 50%, at least 60%, at least 70%, at least 80%, at least 90%, or at least 95% of the length of the reference sequence. The actual comparison of two sequences can be done by a well-known method (e.g. using a mathematical algorithm). A non-limiting example of such a mathematical algorithm is given in Karlin, S. and Altschul, S., Proc. Natl. Acad. Sci. USA, 90-5873-5877 (1993). This algorithm is incorporated into the NBLAST and XBLAST programs (version 2.0), as described in Altschul, S. et al., Nucleic Acids Res., 25:3389-3402 (1997). When the BLAST and Gapped BLAST programs are used, any relevant parameters of the respective program (e.g., NBLAST) can be used. For example, the parameters for sequence comparison can be set to score = 100, word length = 12, or can be changed (e.g., W=5 or W=20).

In some embodiments, the nucleic acids can include analogs (e.g., phosphorothioates, phosphoramidates, methyl phosphonate, chiral methyl phosphonates, and 2-O-methylribonucleotide), modified nucleic acids (e.g., modified backbone residues or linkages), nucleic acids combined with carbohydrates, lipids, polypeptides, or other materials, or peptide nucleic acids (PNA) (e.g., chromatins, ribosomes, and transcripts). In some embodiments, the nucleic acids can include nucleic acids of various structures, such as A DNA, B DNA, Z DNA, siRNA, tRNA, and ribozymes. In some embodiments, the nucleic acids may be naturally or non-naturally polymorphic, e.g., they have one or more sequence differences, e.g., additions, deletions, and/or substitutions compared to a reference sequence. In some embodiments, the reference sequence may be based on publicly available information, such as the University of California, Santa Cruz Human Genome Browser Gateway (UC Santa Cruz Human Genome Browser Gateway) (genome.ucsc.edu/cgi-bin/hgGateway) or the NCBI website (www.ncbi.nlm.nih.gov). In some embodiments, the reference sequence can be determined by a practitioner of the present disclosure using methods well known in the art, for example, by sequencing the reference nucleic acid.

### Vector

In another aspect of the present invention, a vector is provided. In some embodiments, the vector comprises the recombinant nucleic acid herein. In some embodiments, the vector is a viral vector. In some embodiments, the viral vector is an adeno-associated virus vector.

In some embodiments, the viral vector includes, but is not limited to, one or more of adeno-associated virus serotypes AAV1, AAV2, AAV3, AAV4, AAV5, AAV6, AAV7, AAV8, AAV9 , AAVrh10, AAV2.7m8, AAV-DJ, AAV-PHP.B, AAV-PHP.S, and AAV-PHP.eB.

Adeno-associated virus (AAV) is a small virus that infect humans and some other primate species. The composition disclosed herein comprises the genome of adeno-associated virus (AAV) or a derivative thereof.

The AAV genome is a polynucleotide sequence encoding the functions required to produce AAV virions. These functions include those that play a role in the replication and packaging cycle of AAV in the host cell, involving encapsulation of the AAV genome into AAV virions. The naturally occurring AAV virus is replication-defective and completes the replication and packaging cycle relying on the provision of trans helper functions. Therefore, the AAV genome of the vector of the present invention is generally replication-defective.

The AAV genome may be in a single-stranded form (forward or reverse), or alternatively in a double-stranded form. The use of a double-stranded form allows for bypassing the DNA replication step in the target cell, thus accelerating the transgene expression.

The AAV genome can be derived from any naturally occurring AAV serotype or isolate or clade. Therefore, the AAV genome may be a complete genome of a naturally occurring AAV virus. As is known to the skilled person, the AAV viruses present in nature can be classified according to various biological systems.

Typically, the AAV viruses are referred to according to their serotypes. A serotype corresponds to a variant subspecies of AAV that has unique reactivity due to the expression profile of antigens on its capsid surface which can be used to distinguish the variant subspecies from other variant subspecies. Typically, viruses of a particular AAV serotype do not cross-react efficiently with neutralizing antibodies specific for any other AAV serotype. AAV serotypes include AAV1, AAV2, AAV3, AAV4, AAV5, AAV6, AAV7, AAV8, AAV9, AAV10, AAV11, AAV12, AAV13, AAV14, AAV15, and AAV16, as well as recombinant serotypes recently identified from primate brains, such as Rec2 and Rec3.

In some embodiments, the AAV serotype is AAV2; and in some embodiments, the serotype is AAV4, AAV5, or AAV8. They efficiently transduce the tissue in the eyes, such as the retinal pigment epithelium. In some embodiments, the AAV serotype used may be an AAV serotype other than AAV4. Reviews of AAV serotypes can be found in Choi et al. (Curr Gene Ther. 2005; 5(3); 299-310) and Wu et al. (Molecular Therapy. 2006; 14(3), 316-327). The sequence of AAV genome or AAV genome element comprising an ITR sequence and rep or cap gene used herein can be derived from the AAV full genome sequences under the following accession numbers: Adeno-associated virus 1 NC_002077, AF063497; Adeno-associated virus 2 NC_001401; Adeno-associated virus 3 NC_001729; Adeno-associated virus 3B NC_001863; Adeno-associated virus 4 NC_001829; Adeno-associated virus 5 Y18065, AF085716; Adeno-associated virus 6 NC_001862; Avian AAV ATCC VR-865 AY186198, AY629583, NC_004828; Avian AAV strain DA-1 NC_0062 63. AY629583; and bovine AAV NC_005889, AY388617.

AAV viruses may also be referred to on the basis of clades or clones. This refers to the phylogenetic relationships of naturally occurring AAV viruses and generally refers to the phylogenetic group of AAV viruses that can be traced back to a common ancestor and includes all their descendants. Furthermore, the AAV viruses can be referred to in terms of specific isolates (i.e., the genetic isolates of a particular AAV virus found in nature). The term genetic isolate describes a population of AAV viruses that has undergone limited genetic intermingling with other naturally occurring AAV viruses, thereby defining identifiable distinct populations at the genetic level.

Examples of AAV clades and isolates useful herein include, but are not limited to: Clade A: AAV1 NC_002077, AF063497, AAV6 NC_001862, Hu. 48 AY530611, Hu 43 AY530606, Hu 44 AY530607, Hu 46 AY530609; Clade B: Hu. 19 AY530584, Hu. 20 AY530586, Hu 23 AY530589, Hu22 AY530588, Hu24 AY530590, Hu21 AY530587, Hu27 AY530592, Hu28 AY530593, Hu 29 AY530594, Hu63 AY5 30624, Hu64 AY530625, Hu13 AY530578, Hu56 AY530618, Hu57 AY530619, Hu49 AY530612, Hu58 AY530620, Hu34 AY530598, Hu35 AY530599, AAV2 NC_001401, Hu45 AY530608, Hu47 AY530610, Hu51 AY530613, Hu52 AY530614, Hu T41 AY695378, Hu S17 AY695376, Hu T88 AY695375, Hu T71 AY695374, Hu T70 AY695373, Hu T40 AY695372, Hu T32 AY695371, Hu T17 AY695370, Hu LG15 AY695377; Clade C: Hu9 AY530629, Hu10 AY530576, Hu11 AY530577, Hu53 AY530615, Hu55 AY530617, Hu54 AY530616, Hu7 AY530 628, Hu18 AY530583, Hu15 AY530580, Hu16 AY530581, Hu25 AY530591, Hu60 AY530622, Ch5 AY243021, Hu3 AY530595, Hu1 AY530575, Hu4 AY530602 Hu2, AY530585, Hu61 AY530623; Clade D: Rh62 AY530573, Rh48 AY530561, Rh54 AY530567, Rh55 AY530568, Cy2 AY243020, AAV7 AF513851, Rh35 AY243000, Rh37 AY242998, Rh36 AY242999 , Cy6 AY243016, Cy4 AY243018, Cy3 AY243019, Cy5 AY243017, Rh13 AY243013; Clade E: Rh38 AY530558, Hu66 AY530626, Hu42 AY530605, Hu67 AY530627, Hu40 AY5 30603, Hu41 AY530604, Hu37 AY530600, Rh40 AY530559, Rh2 AY243007, Bb1 AY243023, Bb2 AY243022, Rh10 AY243015, Hu17 AY530582, Hu6 AY530621, Rh25 AY530557, Pi2 AY530554, Pi1 AY530553, Pi3 AY530555, Rh57 AY530569, Rh50 AY530563, Rh 49 AY530562, Hu39 AY530601, Rh58 AY530570, Rh61 AY530572, Rh52 AY530565, Rh53 AY530566, Rh51 AY530564 , Rh64 AY530574, Rh43 AY530560, AAV8 AF513852, Rh8 AY242997, Rh1 AY530556; and Clade F: Hu14 (AAV9) AY530579, Hu31 AY530596, Hu32 AY530597, clonal isolate AAV5 Y18065, AF085716, AAV 3 NC_001729, AAV 3B NC_001863, AAV4 NC_001829 , Rh34 AY243001, Rh33 AY243002, Rh32 AY243003.

Appropriate AAV serotypes, clades, clones or isolates for use herein can be selected by skilled artisans based on their common knowledge. For example, the AAV5 capsid is shown to efficiently transduce the primate cone photoreceptors, as demonstrated by the successful correction of inherited color vision defects (Mancuso et al., Nature 2009, 461:784-7).

It is to be understood, however, that the present invention also encompasses the use of AAV genomes of other serotypes that may not yet be identified or characterized. The AAV serotype determines the tissue specificity (or tropism) of AAV virus infection. Therefore, preferred AAV serotypes of AAV viruses to be administered to patients according to the present invention are those with natural tropism or high infection rates for intraocular target cells in the LHON. Thus, the AAV serotype of the AAV virus for administration to a patient may be a serotype that infects cells of the neurosensory retina and retinal pigment epithelium.

Typically, the AAV genome of a naturally derived AAV serotype, isolate, or clade contains at least one inverted terminal repeat (ITR). The ITR sequence acts in cis to provide a functional origin of replication and allows for the integration and excision of the vector from the cellular genome. The AAV genome also typically contains a packaging gene, such as rep and/or cap gene encoding the packaging function of AAV virions. The rep gene encodes one or more of the proteins Rep78, Rep68, Rep52 and Rep40 or variants thereof. The cap gene encodes one or more capsid proteins, such as VP1, VP2, and VP3, or variants thereof. These proteins make up the capsid of the AAV virion. Capsid variants are discussed below.

A promoter will be operably linked to each packaging gene. Specific examples of such promoters include the p5, p19 and p40 promoters (Laughlin et al., 1979, PNAS, 76:5567-5571). For example, the p5 and p19 promoters are commonly used in the expression of the rep gene, and the p40 promoter is commonly used in the expression of the cap gene.

As noted above, the AAV genome used in the vector herein may therefore be a complete genome of a naturally occurring AAV virus. For example, a vector containing a complete AAV genome can be used to prepare the AAV virus in vitro. However, although such a vehicle can in principle be administered to a patient, this rarely occurs in practice. Preferably, the AAV genome will be derivatized for the purpose of administration to patients. Such derivatization is standard in the art, and the present invention contemplates the use of any known AAV genomic derivative as well as derivatives that can be produced by techniques known in the art. The derivatization of AAV genomes and AAV capsids is reviewed by Coura and Nardi (Virology Journal, 2007, 4:99) and Choi et al. and Wu et al., supra.

Derivatives of the AAV genome include any truncated or modified form of the AAV genome. Typically, the AAV genome can be significantly truncated to contain a minimal viral sequence while the functionality described above is still retained. This is preferred for the sake of safety, to reduce the risk of recombination of the vector with the wild-type virus and to avoid a cellular immune response induced due to the presence of a viral genetic protein in the target cells.

Typically, the derivative will contain at least one inverted terminal repeat (ITR), preferably more than one ITR, such as two or more ITRs. One or more ITRs can be derived from AAV genomes with different serotypes, or can be chimeric or mutant ITRs. A preferred mutant ITR is one in which trs (terminal resolution site) is deleted. This deletion allows the genome to continue replicating to produce a single-stranded genome containing coding and complementary sequences, that is, a self-complementary AAV genome. This allows for bypassing the DNA replication step in the target cell, thus accelerating the transgene expression.

The one or more ITRs will preferably flank the polynucleotide sequence of the coding sequence at either end. One or more ITRs are preferably included to assist the vector herein in forming a concatemer in the nucleus of the host cell, for example after the conversion of a single-stranded vector DNA to a double-stranded DNA by the DNA polymerase in the host cell. The formation of such episomal concatemers protects the vector construct during the life of the host cell, allowing for prolonged expression of the transgene in vivo.

In preferred embodiments, the ITR element will be the only sequence retained in the derivative from the native AAV genome. Therefore, the derivative will preferably not contain the rep and/or cap genes of the native genome as well as any other sequences of the native genome. This is preferred for the reasons stated above and also reduces the likelihood of the vector integrating into the host cell genome. Furthermore, reducing the size of the AAV genome allows for increased flexibility in incorporating other sequence elements (such as regulatory elements) in the vector in addition to the transgene.

Regarding the AAV2 genome, the following moieties can therefore be removed in the derivative herein: one inverted terminal repeat (ITR) sequence, the replication (rep) gene and the capsid (cap). However, in some embodiments, including in-vitro embodiments, the derivative may additionally comprise one or more of the rep gene and/or cap gene or other viral sequences of the AAV genome. The naturally occurring AAV virus is integrated at a high frequency at a specific site on human chromosome 19 and shows a negligible random integration frequency, allowing for the retention of the integration capacity in the vector tolerable in a therapeutic context.

When the derivative genome contains a gene encoding the capsid protein, namely VP1, VP2 and/or VP3, the derivative may be a chimeric, shuffled or capsid modified derivative of one or more naturally occurring AAV viruses. In particular, the present invention encompasses the provision of a capsid protein sequence, i.e. pseudotype, from a different AAV serotype, clade, clone or isolate in the same vector.

Typically, the chimeric, shuffled or capsid modified derivative will be selected to provide one or more desired functions to the virus. Accordingly, these derivatives may exhibit increased gene delivery efficiency, reduced immunogenicity (humoral or cellular), altered tropism range and /or improved targeting to specific cell types, compared with AAV viruses comprising a naturally occurring AAV genome (such as, the genome of AAV2). The increased gene delivery efficiency can be achieved by: improving the receptor or co-receptor binding at the cell surface, improving the internalization, improving the transport within the cell and into the nucleus, improving the uncoating of virions, and improving the conversion of a single-stranded genome to a double-stranded form. The increased efficiency may also be associated with the altered tropism range or targeting to specific cell populations (whereby the dosage of the vector will not be diluted by administration to an unwanted tissue).

The chimeric capsid proteins include those produced by recombination between two or more capsid coding sequences of naturally occurring AAV serotypes. This can be performed, for example, by a marker rescue approach, in which a non-infectious capsid sequence of one serotype is co-transfected with a capsid sequence of a different serotype, and a capsid sequence with the desired properties is selected by directed selection. The capsid sequences of different serotypes can be altered by homologous recombination within the cell to produce a new chimeric capsid protein.

The chimeric capsid proteins also include those produced by engineering the capsid protein sequence to transfer a specific capsid domain, surface loops, or specific amino acid residues between two or more capsid proteins, such as between two or more capsid proteins of different serotypes.

The shuffled or chimeric capsid proteins can also be produced by DNA shuffling or by error-prone PCR. The hybrid AAV capsid gene can be produced by randomly fragmenting sequences of related AAV genes, such as those encoding capsid proteins of multiple different serotypes, and subsequently reassembling these fragments in a self-priming polymerase reaction, in which the process can also lead to the crossover of regions of sequence homology. The hybrid AAV gene library generated by shuffling the capsid genes of several serotypes can be screened to identify the viral clones with the desired functions. Similarly, error-prone PCR can be used to randomly mutate the AAV capsid genes to produce a diverse library of variants that can then be screened for desired properties.

The sequence of the capsid gene can also be genetically modified to introduce specific deletions, substitutions or insertions relative to the native wild-type sequence. In particular, the capsid gene can be modified by inserting a coding sequence of an irrelevant protein or peptide within the open reading frame of the capsid coding sequence or at the N-terminus and/or C-terminus of the capsid coding sequence.

The irrelevant protein or peptide may be one that advantageously acts as a ligand for a specific cell type, to confer improved binding to the target cells or increasing the specificity of the virus targeting a specific cell population. Examples may include the use of RGD peptides to block the uptake by the retinal pigment epithelium and thus enhance the transduction in surrounding retinal tissue (Cronin et al., 2008 ARVO Abstract: D1048). The irrelevant protein can also be a protein that serves as a part of the production process to facilitate the purification of virions, i.e., an epitope or an affinity tag. The insertion site is usually chosen so as not to interfere with other functions of the virions, such as the internalization and transport of the virions. A skilled person can identify suitable insertion sites based on their common knowledge. Specific sites are disclosed in Choi et al., supra.

The present invention further contemplates providing the sequence of the AAV genome in other order and configurations than the sequence of the native AAV genome. The present invention also encompasses the replacement of one or more AAV sequences or genes with the sequence from another virus or with a chimeric gene composed of sequences from more than one virus. Such chimeric genes may consist of sequences from two or more related viral proteins from different viral species.

For the sake of clarity, the present invention also provides a AAV viral virion comprising the vector of the present invention. The AAV virion as used herein include a transcapsidated form in which an AAV genome or derivative having the ITR of one serotype are packaged in a capsid of a different serotype. The AAV virion herein also includes a mosaic form in which a mixture of unmodified capsid proteins from two or more different serotypes constitutes the viral envelope. The AAV virion also includes chemically modified form with a ligand adsorbed onto the capsid surface. For example, such ligands may include antibodies targeting specific cell surface receptors.

The present invention further provides a host cell comprising the vector or AAV virion herein. All of the above additional constructs can be provided in the host cells as plasmids or other episomal elements, or alternatively one or more constructs can be integrated into the genome of the host cell.

In these aspects, the present invention provides a method for producing the AAV viruses of the present invention. The method includes providing a vector comprising an adeno-associated virus (AAV) genome or a derivative thereof and a polynucleotide sequence encoding a relevant protein product (such as an antibody) in a host cell, and providing a means for replicating the vector and assembling the vector into AAV virions. Preferably, the method includes providing a vector comprising a derivative of the AAV genome, a polynucleotide sequence encoding a protein/antibody, and one or more additional genetic constructs encoding AAV and/or helper virus functions. Typically, the derivative of the AAV genome includes at least one ITR. Optionally, the method further includes the step of purifying the assembled virions. Additionally, the method may include the step of preparing virions for therapeutic use.

### Pharmaceutical preparations, uses, routes of administration and effective doses

In another aspect of the present invention, a pharmaceutical preparation is provided, which is characterized by containing the aforementioned recombinant nucleic acid, vector or viral virion encoding an anti-VEGF antibody. In some embodiments, the pharmaceutical preparation further includes a pharmaceutically acceptable carrier and/or excipient. In some embodiments, the pharmaceutical preparation is a liquid preparation.

Another aspect of the present invention provides use of the recombinant nucleic acid, vector, viral virion or pharmaceutical preparation as described herein in the treatment of eye diseases. In some embodiments, the eye disease is choroidal neovascular disease. In some embodiments, the choroidal neovascular disease is age-related macular degeneration (AMD) or diabetic retinopathy (DR). In some embodiments, the diabetic retinopathy is diabetic macular edema (DMO).

In some embodiments, the administration dosage of the recombinant nucleic acid, the vector, the virion or the pharmaceutical preparation as descried herein is provided, which is a therapeutically effective amount. The dosage refers to the total dose delivered to a subject during treatment, or the amount delivered upon administration of a single unit (or multiple units or divided doses). The dosage can be expressed in genomic copies (GC) of the nucleic acid sequence. The dosage can also be expressed in terms of viral virions. In some embodiments, the suitable dosage ranges from about 1.0 x 10⁶ GC to about 1.0 x 10¹⁵ GC of viral virions, including all integer or fractional amounts within the range. In some embodiments, the administered dosage is at least 1 x 10⁶, 2 x 10⁶, 3 x 10⁶, 4 x 10⁶, 5 x 10⁶, 6 x 10⁶, 7 x 10⁶, 8 x 10⁶, or 9 x 10⁶ GC per administration, including all integer or fractional amounts within the range. In some embodiments, the administered dosage is at least 1 x 10⁷, 2 x 10⁷, 3 x 10⁷, 4 x 10⁷, 5 x 10⁷, 6 x 10⁷, 7 x 10⁷, 8 x 10⁷ or 9 x 10⁷ GC per administration, including all integer or fractional amounts within the range. In some embodiments, the administered dosage is at least 1 x 10⁸, 2 x 10⁸, 3 x 10⁸, 4 x 10⁸, 5 x 10⁸, 6 x 10⁸, 7 x 10⁸, 8 x 10⁸ or 9 x 10⁸ GC per administration, including all integer or fractional amounts within the range. In some embodiments, the administered dosage is at least 1 x 10⁹, 2 x 10⁹, 3 x 10⁹, 4 x 10⁹, 5 x 10⁹, 6 x 10⁹, 7 x 10⁹, 8 x 10⁹ or 9 x 10⁹ GC per administration, including all integer or fractional amounts within the range. In some embodiments, the administered dosage is at least 1 x 10¹⁰, 2 x 10¹⁰, 3 x 10¹⁰, 4 x 10¹⁰, 5 x 10¹⁰, 6 x 10¹⁰, 7 x 10¹⁰, 8 x 10¹⁰ or 9 x 10¹⁰ GC per administration, including all integer or fractional amounts within the range. In some embodiments, the administered dosage is at least 1 x 10¹¹, 2 x 10¹¹, 3 x 10¹¹, 4 x 10¹¹, 5 x 10¹¹, 6 x 10¹¹, 7 x 10¹¹, 8 x 10¹¹ or 9 x 10¹¹ GC per administration, including all integer or fractional amounts within the range. In some embodiments, the administered dosage is at least 1 x 10¹², 2 x 10¹², 3 x 10¹², 4 x 10¹², 5 x 10¹², 6 x 10¹², 7 x 10¹², 8 x 10¹² or 9 x 10¹² GC per administration, including all integer or fractional amounts within the range. In some embodiments, the administered dosage is at least 1 x 10¹³, 2 x 10¹³, 3 x 10¹³, 4 x 10¹³, 5 x 10¹³, 6 x 10¹³, 7 x 10¹³, 8 x 10¹³ or 9 x 10¹³ GC per administration, including all integer or fractional amounts within the range. In some embodiments, the administered dosage is at least 1 x 10¹⁴, 2 x 10¹⁴, 3 x 10¹⁴, 4 x 10¹⁴, 5 x 10¹⁴, 6 x 10¹⁴, 7 x 10¹⁴, 8 x 10¹⁴ or 9 x 10¹⁴ GC per administration, including all integer or fractional amounts within the range. In some embodiments, the administered dosage is at least 1 x 10¹⁵, 2 x 10¹⁵, 3 x 10¹⁵, 4 x 10¹⁵, 5 x 10¹⁵, 6 x 10¹⁵, 7 x 10¹⁵, 8 x 10¹⁵ or 9 x 10¹⁵ GC per administration, including all integer or fractional amounts within the range. In some embodiments, for use in human, the dosage may range from 1 x 10⁶ to about 1 x 10¹³ GC per administration, including all integer or fractional amounts within the range. In some embodiments, for use in human, the dosage may range from 1 x 10¹⁰ to about 1 x 10¹² GC per administration, including all integer or fractional amounts within the range. Further administration and dosage can be determined by the attending physician.

Depending on the size of the area to be treated, the virus titer used, the route of administration and the desired effect of the approach, These administrations may be given in a carrier, an excipient, or a buffer preparation of various volumes ranging from about 25 to about 1000 microliters, including all numbers within the range. In some embodiments, the volume administered is at least about 25 µl. In some embodiments, the volume is about 50 µl. In some embodiments, the volume is about 75 µl. In some embodiments, the volume is about 100 µl. In some embodiments, the volume is about 125 µl. In some embodiments, the volume is about 150 µl. In some embodiments, the volume is about 175 µl. In some embodiments, the volume is about 200 µl. In some embodiments, the volume is about 225 µl. In some embodiments, the volume is about 250 µl. In some embodiments, the volume is about 275 µl. In some embodiments, the volume is about 300 µl. In some embodiments, the volume is about 325 µl. In some embodiments, the volume is about 350 µl. In some embodiments, the volume is about 375 µl. In some embodiments, the volume is about 400 µl. In some embodiments, the volume is about 450 µl. In some embodiments, the volume is about 500 µl. In some embodiments, the volume is about 550 µl. In some embodiments, the volume is about 600 µl. In some embodiments, the volume is about 650 µl. In some embodiments, the volume is about 700 µl. In some embodiments, the volume is between about 700 and 1000 µl.

The nucleic acid, vector, or viral virion of the present disclosure may be administered as pharmaceutical preparations, including those pharmaceutical preparations suitable for oral (including buccal and sublingual), rectal, nasal, topical, transdermal, pulmonary, vaginal, suppository, or parenteral (including intraocular, intravitreal, intramuscular, intraarterial, intrathecal, intradermal, intraperitoneal, subcutaneous and intravenous) administration or in a form suitable for administration by nebulization, inhalation or insufflation. General information on drug delivery systems can be found in Ansel et al., Pharmaceutical Dosage Forms and Drug Delivery Systems (Lippencott Williams & Wilkins, Baltimore Md. (1999).

In some embodiments, the nucleic acid, vector, viral virion, or pharmaceutical preparation of the present disclosure can be administered via any available delivery method or route. Preferably, the mode of delivery is topical administration. All routes of topical administration to the eye that can be considered include topical, subconjunctival, periocular, retrobulbar, subtenon, intraocular, intravitreal, intracameral, subretinal, and suprachoroidal administration. Systemic or parenteral administration is possible, including, but not limited to, intravenous, subcutaneous, and oral delivery. The most preferred methods of administration are intravitreal or subtenon injection of solutions or suspensions; intravitreal or subtenon placement of bioerodible or nonbioerodible devices (implants); or topical intraocular administration of solutions or suspensions. In a preferred embodiment, the method of administration is administration as a solution, suspension or gel through a posterior juxtascleral route. In another preferred embodiment, the method of administration is administration as a bioerodible implant through an intravitreal route.

In some embodiments, the nucleic acid, vector, viral virion or pharmaceutical preparation of the present disclosure is administered by intraocular injection, preferably intravitreal injection.

In various embodiments, the pharmaceutical composition include a carrier and an excipient (including, but not limited to, a buffer, a carbohydrate, mannitol, a polypeptide, an amino acid, an antioxidant, a bacteriostatic agent, a chelating agent, a suspending agent, a thickener and/or a preservative), water, an oil (including those derived from petroleum, animals, plants or produced synthetically, such as peanut oil, soybean oil, mineral oil, and sesame oil, etc.), a saline solution, an aqueous dextrose and glycerol solution, a flavoring, a colorant, an anti-sticking agent and other acceptable additives, adjuvants or binders, and other pharmaceutically acceptable auxiliary substances used to approximate the physiological conditions, such as a pH buffer, an isotonic regulator, an emulsifier, and a wetting agent, etc. Examples of excipients include starch, glucose, lactose, sucrose, gelatin, malt, rice, flour, chalk, silica gel, sodium stearate, glyceryl monostearate, talc, sodium chloride, skimmed milk powder, glycerin, propylene, ethylene glycol, water, and ethanol, etc. In some embodiments, the pharmaceutical preparation is substantially free of preservatives. In other embodiments, the pharmaceutical preparation may contain at least one preservative. General methods on the dosage forms can be found in Ansel et al., Pharmaceutical Dosage Forms and Drug Delivery Systems (Lippencott, Williams ,& Wilkins, Baltimore Md. (1999)). It will be appreciated that although the composition of the present disclosure can be administered with any suitable carrier known to those of ordinary skill in the art, the type of the carrier may vary depending on the mode of administration. The compound can also be encapsulated within liposome using well known techniques. Biodegradable microspheres may also be used as a carrier for the pharmaceutical composition of the present disclosure. Suitable biodegradable microspheres are disclosed, for example, in U.S. Patent Nos. 4,897,268, 5,075,109, 5,928,647, 5,811,128, 5,820,883, 5,853,763, 5,814,344, and 5,942,252. The compound can be administered in the form of liposomes or microspheres (or microparticles). Methods for preparing liposomes and microspheres for administration to subjects are well known to those skilled in the art. U.S. Patent No. 4,789,734 (the disclosure of which is incorporated herein by reference) describes a method for encapsulating a biological material into liposome. Basically, the material is dissolved in an aqueous solution, appropriate phospholipid and lipid are added, if needed, a surfactant is added, and the material is dialyzed or sonicated if necessary. A review of known methods is given in G. Gregoriadis, Chapter 14, "Liposomes," Drug Carriers in Biology and Medicine, available at page 2. sup. 87-341 (Academic Press, 1979).

Microspheres formed from polymers or polypeptides are well known to those skilled in the art and can be adapted to pass through the gastrointestinal tract directly into the bloodstream. Alternatively, a compound can be incorporated and microspheres or compositions of microspheres are implanted for slow release over a period of time ranging from days to months. See, for example, U.S. Patent Nos. 4,906,474, 4,925,673, and 3,625,214 and Jein, TIPS 19:155-157 (1998), the disclosures of which are incorporated herein by reference.

The concentration of the drug can be adjusted, the pH of the solution is buffered and the solution is isotonically adjusted so as to be compatible with intraocular or intravitreal injection.

The compound of the present disclosure can be prepared into a sterile solution or suspension in a suitable vehicle. The pharmaceutical composition can be sterilized by a conventional, well-known sterilization technique, or can be aseptically filtered. The resulting aqueous solution may be packaged for use as it is or may be lyophilized and the lyophilized preparation is reconstituted in a sterile solution prior to administration. Suitable preparations and additional carriers are described in Remington "The Science and Practice of Pharmacy" (20th Ed., Lippincott Williams & Wilkins, Baltimore MD), and the teachings of the literature are incorporated in their entirety by reference.

The preparation, or a pharmaceutically acceptable salt thereof, can be provided alone or in combination with one or more other agents or one or more other forms. For example, the preparation may contain one or more agents in specific proportions, depending on the relative potency and intended indication of each agent. For example, in a composition for targeting two different host targets, and where the potency is similar, an approximately 1:1 ratio of agents may be used. The two forms can be prepared together in the same dosage unit, for example, into a cream, a suppository, a tablet, a capsule, an aerosol spray or a packet of powder to be dissolved in a beverage. Alternatively, each form can be prepared into separate units, such as two creams, two suppositories, two tablets, two capsules, a tablet and a liquid for dissolving the tablet, two aerosol sprays, or a packet of powder and a liquid for dissolving the powder, and so on.

The term "pharmaceutically acceptable salt" means those salts that retain the biological effectiveness and properties of the agents used in the present disclosure and are not biologically or otherwise undesirable. Typical salts are those in the form of inorganic ions (such as sodium, potassium, calcium, and magnesium ions, etc). Such salts include salts with inorganic or organic acids such as: hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid or maleic acid. Additionally, if an agent contains a carboxyl group or other acidic groups, the agent can be converted into a pharmaceutically acceptable addition salt with an inorganic or organic base. Examples of suitable bases include sodium hydroxide, potassium hydroxide, ammonia, cyclohexylamine, dicyclohexylamine, ethanolamine, diethanolamine, triethanolamine, and the like.

The pharmaceutically acceptable esters or amides means those esters or amides that retain the biological effectiveness and properties of the agents used in the present disclosure and are not biologically or otherwise undesirable. Typical esters include ethyl ester, methyl ester, isobutyl ester, and ethylene glycol ester, etc. Typical amides include unsubstituted amides, alkyl amides, dialkyl amides, and the like.

In some embodiments, the preparation can be administered in combination with one or more other compounds, forms, and/or agents (e.g., as described above). A pharmaceutical composition comprising one or more other active agents can be prepared to contain certain molar ratios. For example, the first active agent and other active agents at a molar ratio of about 99:1 to about 1:99 can be used. In some subsets of embodiments, the molar ratio of the first active agent:other active agents ranges from about 80:20 to about 20:80, from about 75:25 to about 25:75, from about 70:30 to about 30 :70, from about 66:33 to about 33:66, from about 60:40 to about 40:60, about 50:50, and from about 90:10 to about 10:90. The molar ratio of the first active agent:other active agents can be about 1:9, and about 1:1 in some embodiments. The two agents, forms and/or compounds can be prepared together in the same dosage unit, for example, into a cream, a suppository, a tablet, a capsule, or a packet of powder to be dissolved in a beverage. Alternatively, each form, form and/or compound can be prepared into separate units, such as two creams, two suppositories, two tablets, two capsules, a tablet and a liquid for dissolving the tablet, two aerosol sprays, or a packet of powder and a liquid for dissolving the powder, and so on.

The agent and/or combination of agents can be administered with other agents if necessary or needed. The choice of agents that can be co-administered with the agent and/or combination of agents of the present disclosure can be based, at least in part, on the condition being treated.

The agent (or a pharmaceutically acceptable salt, ester or amide thereof) can be administered as it is or in the form of a pharmaceutical composition in which the active agent is blended or mixed with one or more pharmaceutically acceptable carriers. As used herein, the pharmaceutical composition can be any composition prepared for administration to a subject. The pharmaceutical composition for use in accordance with the present disclosure can be prepared in a conventional manner using one or more physiologically acceptable carriers, including, for example, an excipient, a diluent and/or an auxiliary that facilitate the processing of the active agent into a preparation suitable for administration. Proper preparations depend, at least in part, on the route of administration used. The agent useful in the present disclosure or a pharmaceutically acceptable salt, ester or amide thereof can be delivered to a subject through a variety of routes or modes of administration, including oral, buccal, topical, rectal, transdermal, transmucosal, subcutaneous, intravenous, intraocular, intravitreal and intramuscular application, and by inhalation.

In some embodiments, an oily or non-aqueous solvent can be used to bring the agent into a solution due to, for example, the presence of a large lipophilic moiety. Alternatively, an emulsion, suspension or other preparations, such as a liposomal preparation, can be used. Regarding the liposomal preparation, any known method for preparing liposomes for treating a condition can be used. See, for example, Bangham et al., J. Mol. Biol. 23: 238-252 (1965) and Szoka et al., Proc. Natl Acad. Sci. USA 75: 4194-4198 (1978), which is incorporated herein by reference. The ligand can also be attached to the liposome to direct the composition to a specific site of action. The agent of the present disclosure may also be incorporated into a food product such as cream cheese, butter, salad dressing, or ice cream to promote the dissolution, administration, and/or compliance in certain subject populations.

The compound of the present disclosure can be prepared for parenteral administration (e.g., by injection, such as intraocular or intravitreal injection) and can be presented in a unit dosage form with the added preservative in an ampoule, a prefilled syringe, a small-volume infusion, or a multi-dose container. The composition can adopt such forms as suspensions, solutions or emulsions in oily or aqueous vehicles, for example, solutions in aqueous polyethylene glycol.

For injectable preparations, the vehicle can be selected from those known in the art to be suitable, including aqueous or oily suspensions, or emulsions, and sesame oil, corn oil, cottonseed oil, or peanut oil, and elixirs, mannitol, dextrose, or sterile aqueous solutions, and similar pharmaceutical vehicles. The preparation can also include a composition in a biocompatible, biodegradable polymer such as poly(lactic-co-glycolic acid). These materials can be prepared into microspheres or nanospheres loaded with drugs, which are further coated or derivatized to provide excellent performance of sustained release. Vehicles suitable for periorbital or intraocular injection include, for example, suspensions of the therapeutic agent in water for injection, liposomes, and vehicles suitable for lipophilic substances. Other vehicles for periocular or intraocular injection are well known in the art.

In some embodiments, the composition is prepared according to conventional procedures into a pharmaceutical composition suitable for intravenous administration to humans. Typically, the composition for intravenous administration is a solution in the form of a sterile isotonic aqueous buffer solution. If necessary, the composition may also contain a solubilizer and a local anesthetic such as lidocaine to reduce the pain at the injection site. Generally, the ingredients are provided, alone or in admixture, in unit dosage form (for example, as a lyophilized powder or anhydrous concentrate) in a closed container (such as an ampoule or sachet) indicating the amount of the active agent. When the composition is to be administered by infusion, the composition can be dispensed from an infusion bottle containing sterile water or saline for pharmaceutical use. Where the composition is administered by injection, ampoules of sterile water or saline for injection may be provided so that the ingredients can be mixed prior to administration.

When administered by injection, the active compound can be prepared into an aqueous solution, particularly a physiologically compatible buffer solution, such as Hanks solution, Ringer's solution or physiological saline buffer. The solution may contain a formulatory agent such as a suspending agent, a stabilizing agent and/or a dispersing agent. Alternatively, the active compound can be in a powder form for reconstitution in a suitable vehicle, such as sterile pyrogen-free water, before use. In some embodiments, the pharmaceutical composition does not contain adjuvants or any other substances added to enhance the immune response stimulated by the peptide. In some embodiments, the pharmaceutical composition includes a substance that inhibits the immune response to the peptide. The preparation methods are known in the art, for example as disclosed in Remington's Pharmaceutical Sciences, latest edition, Mack Publishing Co., Easton P.

In some embodiments, the ocular conditions can be effectively treated with ophthalmic solutions, suspensions, ointments, or inserts containing an agent or combination of agents of the present disclosure. Eye drops can be prepared before use by dissolving the active ingredient in a sterile aqueous solution such as physiological saline and a buffered solution, or by combining a powder composition to be dissolved. Other vehicles known in the art can be used, including, but not limited to, a balanced salt solution, a saline solution, a water-soluble polyether such as polyethylene glycol, polyethylene such as polyvinyl alcohol and povidone, a cellulose derivative such as methyl cellulose and hydroxypropyl methylcellulose, a petroleum derivative such as mineral oil and white petrolatum, an animal fat such as lanolin, an acrylic polymer such as a carboxypolymethylene gel, a vegetable fat such as peanut oil, a polysaccharide such as dextran, and a glycosaminoglycan such as sodium hyaluronate. If necessary, an additive commonly used in eye drops can be added. Such additives include an isotonic agent (e.g., sodium chloride, etc.), a buffering agent (e.g., boric acid, disodium hydrogen phosphate, and sodium dihydrogen phosphate, etc.), a preservative (e.g., benzalkonium chloride, benzyl benzethonium chloride, and chlorobutanol, etc.), and a thickener (for example, a sugar such as lactose, mannitol, and maltose, etc.; for example, hyaluronic acid or a salt thereof such as sodium hyaluronate, and potassium hyaluronate, etc.; for example, mucopolysaccharides such as chondroitin sulfate, etc.; for example, sodium polyacrylate, a carboxyvinyl polymer, cross-linked polyacrylate, polyvinyl alcohol, polyvinylpyrrolidone, methylcellulose, hydroxypropylmethylcellulose, Hydroxyethylcellulose, carboxymethylcellulose, hydroxypropylcellulose or other agents known to those skilled in the art).

The solubility of components in the composition herein can be increased by the surfactant or other suitable co-solvents in the composition. Such cosolvents include Polysorbate 20, Polysorbate 60 and Polysorbate 80; Pluronic F68, Pluronic F-84 and Pluronic P-103, cyclodextrins or other agents known to those skilled in the art. Such co-solvents can be used at a level of about 0.01% to 2% by weight.

The composition of the present disclosure can be packaged in multiple dosage forms. A preservative can be preferably used to prevent microbial contamination during use. Suitable preservatives include: benzalkonium chloride, thimerosal, chlorobutanol, methylparaben, propylparaben, phenethyl alcohol, disodium edetate, sorbic acid, Onamer M or other agents known to those skilled in the art. In the ophthalmic products in the prior art, such preservatives can be used at a level of 0.004% to 0.02%. In the composition of the present application, the preservative (preferably benzalkonium chloride) can be used at a level of from 0.001% to less than 0.01% by weight (e.g., from 0.001 to 0.008% by weight, preferably about 0.005% by weight). A benzalkonium chloride concentration of 0.005% has been found to be sufficient to protect the composition of the present disclosure from microbial attack.

In some embodiments, the agent of the present disclosure is delivered in a soluble form rather than a suspension, which allows for more rapid and quantitative absorption into the site of action. In general, preparations such as gels, creams, lotions, suppositories, and ointments can provide extended exposure to the agent of the present disclosure, and preparations in solutions, such as sprays, can provide more immediate short-term exposure.

It is further contemplated that the compound of the present disclosure can be releasably attached to a biocompatible polymer for use in a sustained-release preparation on, in, or attached to an insert. The insert is used for topical, intraocular, periocular or systemic administration. A biocompatible polymer of controlled release can also be used with a water soluble polymer to form a droppable preparation. Controlled release from a biocompatible polymer such as PLGA microspheres or nanospheres can be used with a preparation suitable for intraocular implantation or injection to achieve sustained-release administration, or any suitable biodegradable and biocompatible polymers can be used.

### Other numbered implementations

Implementation 1. A recombinant nucleic acid, encoding an anti-VEGF heavy chain, the anti-VEGF heaving chain coding sequence comprising a sequence having ≥90%, ≥95%, ≥96%, ≥97%, ≥98%, ≥99%, ≥99.5% or 100% identity to one of the sequences as shown in SEQ ID NOs: 5 and 6 and 23 to 26.

Implementation 2. The recombinant nucleic acid according to Implementation 1, where the amino acid sequence of the anti-VEGF heavy chain comprises a sequence having ≥90%, ≥95%, ≥96%, ≥97%, ≥98%, ≥99%, ≥99.5% or 100% identity to the sequence as shown in SEQ ID NOs: 17 and 18.

Implementation 3. The recombinant nucleic acid according to Implementation 1 or 2, where the sequences of the complementarity determining region 1 (CDR1), the complementarity determining region 2 (CDR2), and the complementarity determining region 3 (CDR3) of the anti-VEGF heavy chain are identical to the sequences as shown in SEQ ID NO: 31, SEQ ID NO: 32, and SEQ ID NO: 33 respectively.

Implementation 4. The recombinant nucleic acid according to any one of Implementations 1 to 3, where the anti-VEGF heavy chain does not include a constant region 2 (CH2) and a constant region 3 (CH3).

Implementation 5. A recombinant nucleic acid, encoding an anti-VEGF light chain, the coding sequence of the anti-VEGF light chain comprising a sequence having ≥90%, ≥95%, ≥96%, ≥97%, ≥98%, ≥99%, ≥99.5% or 100% identity to one of the sequences as shown in SEQ ID NOs: 4 and 19 to 22.

Implementation 6. The recombinant nucleic acid according to Implementation 5, where the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO: 16.

Implementation 7. The recombinant nucleic acid according to Implementation 5 or 6, where the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30 respectively.

Implementation 8. The recombinant nucleic acid according to any one of Implementations 1 to 4, where the recombinant nucleic acid further encodes an anti-VEGF light chain, and the anti-VEGF light chain encoding sequence comprises a sequence having ≥90%, ≥95%, ≥96%, ≥97%, ≥98%, ≥99%, ≥99.5% or 100% identity to one of the sequences as shown in SEQ ID NOs: 4 and 19 to 22.

Implementation 9. The recombinant nucleic acid according to Implementation 8, where the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO: 16.

Implementation 10. The recombinant nucleic acid according to Implementation 9, where the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30 respectively.

Implementation 11. The recombinant nucleic acid according to any one of Implementations 8 to 10, where the combination of SEQ ID NOs: 4 and 19 to 22 with SEQ ID NOs: 5 and 6 and 23 to 26 is a combination selected from Table 1.

Implementation 12. The recombinant nucleic acid according to any one of Implementations 8 to 10, where the combination of SEQ ID NOs: 4 and 19 to 22 with SEQ ID NOs: 5 and 6 and 23 to 26 is a combination selected from Table 3.

Implementation 13. The recombinant nucleic acid according to any one of Implementations 8 to 10, where the coding sequence of the anti-VEGF heavy chain comprises a sequence having ≥99%, ≥99.5% or 100% identity to the sequence as shown in SEQ ID NO: 25, and the coding sequence of the anti-VEGF light chain comprises a sequence having ≥99%, ≥99.5% or 100% identity to the sequence as shown in SEQ ID NO: 21.

Implementation 14. The recombinant nucleic acid according to any one of Implementations 8 to 10, where the coding sequence of the anti-VEGF heavy chain comprises a sequence having ≥99%, ≥99.5% or 100% identity to the sequence as shown in SEQ ID NO: 25, and the coding sequence of the anti-VEGF light chain comprises a sequence having ≥99%, ≥99.5% or 100% identity to the sequence as shown in SEQ ID NO: 21.

Implementation 15. The recombinant nucleic acid according to any one of Implementations 8 to 14, where the coding sequence of the anti-VEGF light chain is located upstream (at 5' end) of the coding sequence of the anti-VEGF heavy chain.

Implementation 16. The recombinant nucleic acid according to any one of Implementations 8 to 15, further comprising a coding sequence of a linker peptide between the coding sequence of the anti-VEGF light chain and the coding sequence of the anti-VEGF heavy chain.

Implementation 17. The recombinant nucleic acid according to Implementation 16, where the amino acid sequence of the linker peptide comprises any one of the sequences as shown in SEQ ID NOs: 14 and 15, or a sequence that differs from any one of the sequences as shown in SEQ ID NOs: 14 and 15 by no more than 1, no more than 2, or no more than 3 amino acids.

Implementation 18. The recombinant nucleic acid according to any one of Implementations 1 to 17, further comprising a promoter and/or an enhancer, where the promoter and/or the enhancer is operably linked to an open reading frame encoding the anti-VEGF light chain and/or the anti-VEGF heavy chain; optionally, the promoter is a chicken β-actin promoter; and optionally, the enhancer is a CMV enhancer.

Implementation 19. The recombinant nucleic acid according to Implementation 18, where the sequence of the promoter/enhancer comprises a sequence having ≥90%, ≥95%, ≥96%, ≥97%, ≥98%, ≥99%, ≥99.5% or 100% identity to the sequence as shown in SEQ ID NO: 1.

Implementation 20. The recombinant nucleic acid according to any one of Implementations 1 to 19, further comprising one or more signal peptide coding sequences, where the signal peptide coding sequence is located upstream (at 5' end) of the coding sequence of the anti-VEGF heavy chain and/or the coding sequence of the anti-VEGF light chain.

Implementation 21. The recombinant nucleic acid according to Implementation 20 or 21, where the signal peptide coding sequence is connected to the 5' end of the anti-VEGF heavy chain; and preferably the signal peptide coding sequence connected to the anti-VEGF heavy chain comprises an amino acid sequence that is ≥90%, ≥95%, ≥96%, ≥97%, ≥98%, ≥99%, or 100% identical to any one of the sequence as shown in SEQ ID NOs: 8 to 13 and 34 and 35, and preferably ≥90%, ≥95%, ≥96%, ≥97%, ≥98%, ≥99%, or 100% identical to the sequence as shown in SEQ ID NO: 34.

Implementation 22. The recombinant nucleic acid according to Implementation 20 or 21, where the signal peptide coding sequence is connected to the 5' end of the anti-VEGF light chain; and preferably the signal peptide coding sequence connected to the anti-VEGF light chain comprises an amino acid sequence that is ≥90%, ≥95%, ≥96%, ≥97%, ≥98%, ≥99%, or 100% identical to any one of the sequence as shown in SEQ ID NOs: 8 to 13 and 34 and 35, and preferably ≥90%, ≥95%, ≥96%, ≥97%, ≥98%, ≥99%, or 100% identical to the sequence as shown in SEQ ID NO: 35.

Implementation 23. The recombinant nucleic acid according to any one of Implementations 1 to 22, further comprising an intron, where optionally, the intron is located between the sequence of the promoter/enhancer and the signal peptide coding sequence.

Implementation 24. The recombinant nucleic acid according to Implementation 23, where the sequence of the intron comprises a sequence that is ≥90%, ≥95%, ≥96%, ≥97%, ≥98%, ≥99%, ≥99.5% or 100% identical to any one of the sequence as shown in SEQ ID NO: 2 and 3.

Implementation 25. The recombinant nucleic acid according to any one of Implementations 1 to 24, further comprising a poly(A) sequence, where the poly(A) sequence is located downstream (at 3' end) of the coding sequence of the anti-VEGF heavy chain and/or the coding sequence of the anti-VEGF light chain; and optionally, the poly(A) sequence is human growth hormone poly(A) sequence.

Implementation 26. The recombinant nucleic acid according to Implementation 25, where the poly(A) sequence comprises a sequence that is ≥90%, ≥95%, ≥96%, ≥97%, ≥98%, ≥99%, ≥99.5%, or 100% identical to the sequence as shown in SEQ ID NO: 7.

Implementation 27. The recombinant nucleic acid according to any one of Implementations 1 to 26, further comprising a woodchuck hepatitis virus post-transcriptional regulatory element (WPRE) sequence, where the WPRE sequence is located between the open reading frame encoding the anti-VEGF light chain and/or the anti-VEGF heavy chain and the poly(A) sequence.

Implementation 28. The recombinant nucleic acid according to Implementation 27, where the WPRE sequence comprises a sequence that is ≥90%, ≥95%, ≥96%, ≥97%, ≥98%, ≥99%, ≥99.5% or 100% identical to the sequence as shown in SEQ ID NO: 27.

Implementation 29. A viral vector, comprising the recombinant nucleic acid according to any one of Implementations 1 to 28.

Implementation 30. The viral vector according to Implementation 29, where the vector is an adeno-associated virus (AAV) vector.

Implementation 31. The viral vector according to Implementation 30, where the serotype of the adeno-associated virus vector is one or more selected from the group consisting of AAV1, AAV2, AAV3, AAV4, AAV5, AAV6, AAV7, AAV8, AAV9, AAVrh10, AAV2.7m8, AAV- DJ, AAV-PHP.B, AAV-PHP.S, and AAV-PHP.eB.

Implementation 32. The viral vector according to Implementation 30 or 31, further comprising an inverted terminal repeat (ITR) of AAV.

Implementation 33. A viral virion, comprising the viral vector according to any one of Implementations 29 to 32.

Implementation 34. The viral virion according to Implementation 33, where the viral virion is an AAV viral virion.

Implementation 35. The viral virion according to Implementation 34, where the serotype of the AAV virion is one or more selected from the group consisting of AAV1, AAV2, AAV3, AAV4, AAV5, AAV6, AAV7, AAV8, AAV9 , AAVrh10, AAV2.7m8, AAV-DJ, AAV-PHP.B, AAV-PHP.S, and AAV-PHP.eB.

Implementation 36. A pharmaceutical preparation, comprising the viral virion according to any one of Implementations 33 to 35, the viral vector according to any one of Implementations 29 to 32, or the recombinant nucleic acid according to any one of Implementations 1 to 28, and a pharmaceutically acceptable carrier or excipient.

Implementation 37. The pharmaceutical preparation according to Implementation 36, where the pharmaceutical preparation is a liquid preparation.

Implementation 38. Use of the viral virion according to any one of Implementations 33 to 35, the viral vector according to any one of Implementations 29 to 32, or the recombinant nucleic acid according to any one of Implementations 1 to 28 in the preparation of medicines for treating eye diseases.

Implementation 39. A method for treating eye diseases, comprising administering to a subject in need thereof the pharmaceutical preparation according to any one of Implementations 36 and 37, the viral virion according to any one of Implementations 33 to 35, the viral vector according to any one of Implementations 29 to 32, or the recombinant nucleic acid according to any one of Implementations 1 to 28.

Implementation 40. The use according to Implementation 38 or the method according to Implementation 39, where the eye disease is choroidal neovascular disease.

Implementation 41. The use or method according to Implementation 40, where the choroidal neovascular disease is age-related macular degeneration or diabetic retinal degeneration; and preferably, the diabetic retinal degeneration is diabetic macular edema.

Implementation 42. The use or method according to any one of Implementations 38 to 41, where the viral virion, the viral vector, the recombinant nucleic acid, or the pharmaceutical preparation is injected intraocularly, and preferably, intravitreally injected.

Implementation 43. The use or method according to any one of Implementations 38 to 42, where the viral virion, the viral vector, or the pharmaceutical preparation are capable of long-lasting expression of the anti-VEGF antibody or an antigen binding fragment thereof in the eye, to prevent the eye diseases caused by VEGF overexpression.

### Examples

### Example 1: Construction and expression screening of viral vectors

Anti-VEGF Fab expression plasmids MDR1, MDR2, MDR3, MDR4, MDR5, MDR6, OPT1, OPT4, OPT5, and OPT6 were constructed.

The pAAV-CBA-MDR (hereinafter referred to as pAAVMDR) viral vector was constructed. The vector genome contains AAV inverted terminal repeat ITR, CBA promoter (chicken β-actin, see SEQ ID NO: 1 for nucleic acid sequence) and MDR target gene sequence. The CMV enhancer that enhances gene expression is added before the CBA promoter. The target gene expression cassette contains a synthetic intron (SEQ ID NO: 2 or SEQ ID NO: 3), a signal peptide (selected from SEQ ID NOs: 8-13 and 34-35), a light chain (selected from SEQ ID NOs: 4 and 19-22), a heavy chain (selected from SEQ ID NOs: 5, 6 and 23-26), a linker peptide (SEQ ID NOs: 14 or SEQ ID NO: 15) separating the light chain and the heavy chain, and a human growth hormone poly(A) sequence (SEQ ID NO: 7) that can increase the stability of the transcript product, and a WPREm element (SEQ ID NO: 27) can also be added. Individual OPT vectors are also constructed in a similar manner. The specific sequence elements used in each expression vector are shown in Table 3:

**Table 3: Constituent elements of each anti-VEGF Fab OPT vector**

| Vector | Synthetic intron | Light chain signal peptide (amino acid sequence) | Light chain | Linker peptide (amino acid sequence) | Heavy chain signal peptide (amino acid sequence) | Heavy chain |
|---|---|---|---|---|---|---|
| OPT1 | SEQ ID NO: 3 | SEQ ID NO: 35 | SEQ ID NO: 19 | SEQ ID NO: 15 | SEQ ID NO: 34 | SEQ ID NO: 23 |
| OPT4 | SEQ ID NO: 3 | SEQ ID NO: 35 | SEQ ID NO: 20 | SEQ ID NO: 15 | SEQ ID NO: 34 | SEQ ID NO: 24 |
| OPT5 | SEQ ID NO: 3 | SEQ ID NO: 35 | SEQ ID NO: 21 | SEQ ID NO: 15 | SEQ ID NO: 34 | SEQ ID NO: 25 |
| OPT6 | SEQ ID NO: 3 | SEQ ID NO: 35 | SEQ ID NO: 22 | SEQ ID NO: 15 | SEQ ID NO: 34 | SEQ ID NO: 26 |

| | | | | | | |
|---|---|---|---|---|---|---|
| *Each expression vector contains AAV inverted terminal repeat ITR, CBA promoter (chicken β-actin)/CMV enhancer, and human growth hormone poly(A) sequence. In addition, some vectors also contain a WPREm element (SEQ ID NO: 27), which is located before the human growth hormone poly(A) sequence. | | | | | | |

### Example 2: Cell transfection, expression and screening of viral vector plasmid, with 1 ug of Lucentis as a standard control

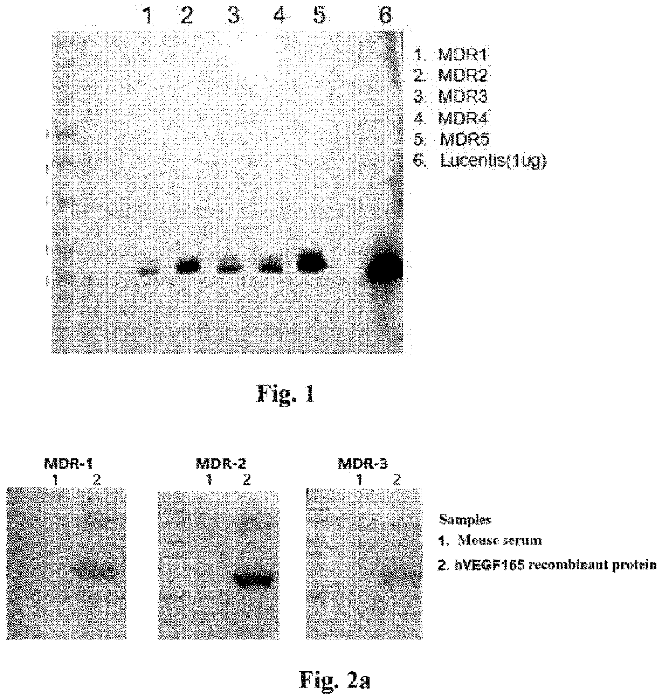

The HEK293 cells were grown to a density of 90% or more. The cells were digested and collected, and inoculated into a 6-well plate at a density of 1 × 10⁶. A blank group (PBS group) and a transfection group (5 samples: transfected with five kinds of pAAVMDR-NFS-kana plasmid) were included. 2.5 ug of each plasmid and PEI were used for transfection. The plasmid:PEI ratio was 1:3. The same amount of PBS was added to the blank group. 72 hrs after transfection, the cell culture supernatant was collected and concentrated to about 0.3 mL in a 10 KD ultrafiltration tube. With 1 ug of Lucentis standard as a reference, 10 µL of each concentrate was separated by SDS-PAGE gel electrophoresis and analyzed by Western blot. After incubation with the secondary antibody Anti-Human Kappa-HRP antibody, development by ECL was performed. As shown in Fig. 1, the samples from left to right are blank control, MDR1, MDR2, MDR3, MDR4, MDR5, and Lucentis respectively. Compared with the Lucentis standard, the anti-VEGF Fab protein band expressed by each pAAVMDR-NFS-kana plasmid is clear, and the molecular weight is consistent with the molecular weight of Lucentis.

### Example 3: Verification of the characteristic binding of Fab expressed by the vector to human VEGF by Western blot with LUCENTIS used as a control

Fig. 2a: 3 samples of mouse serum containing 5 ug of total protein or 1.5 ug of hVEGF165 recombinant protein (purified and prepared by Neuforth) were prepared respectively. The sample was subjected to SDS-PAGE gel electrophoresis and analyzed by Western blot. The supernatant of cells transfected with MDR-1, MDR-2 or MDR-3 plasmid respectively was used as the primary antibody, and the Anti-Human Kappa-HRP antibody was used as the secondary antibody to detect the binding of the expressed anti-VEGF Fab to the antigen hVEGF165.

Fig. 2a shows, from left to right, the results of incubation with the supernatants of cells transfected with MDR1, MDR2 and MDR3 plasmids. As can be seen, the Fab expressed by MDR1, MDR2 and MDR3 specifically binds to human VEGF (hVEGF) and does not bind to any component in mouse serum. This experiment proves that the anti-VEGF Fab protein expressed by the pAAVMDR vector only recognizes human hVEGF165 and does not recognize any component in mouse serum.

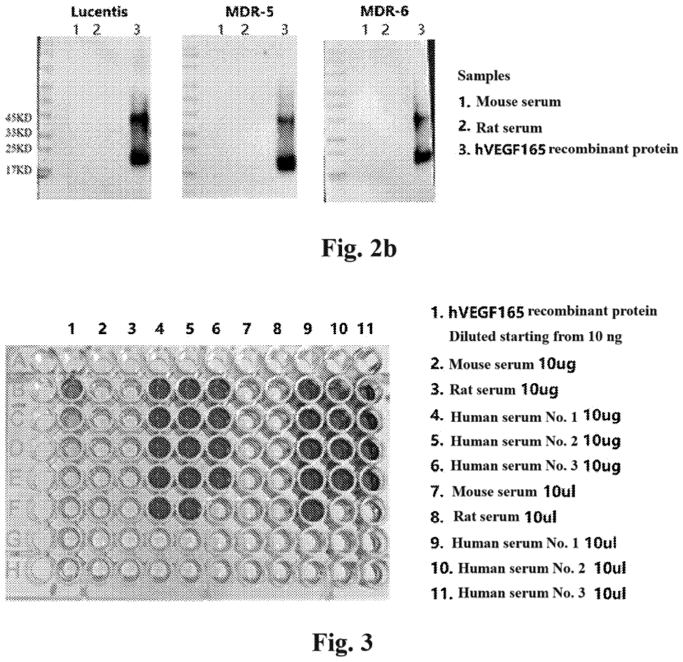

Fig. 2b: Verification of the characteristic binding of Fab expressed by the vector to human VEGF by Western blot with LUCENTIS used as a control.

3 samples of mouse serum containing 5 µg of total protein, 5 µg of rat serum, or 1.5 µg of hVEGF165 recombinant protein (purified and prepared by Neuforth) were prepared respectively. The sample was subjected to SDS-PAGE gel electrophoresis and analyzed by Western blot. The culture supernatant of cells transfected with MDR-5 or MDR-6 plasmids was used as the primary antibody, and Lucentis available from the market was used as a control. The Anti-Human Kappa-HRP antibody was used as the secondary antibody to detect the binding of the expressed anti-VEGF Fab to the antigen hVEGF165.

Fig. 2b shows, from left to right, the results of incubation with Lucentis, the results of incubation with a concentrated sample of cells transfected with MDR5, and the results of incubation with a concentrated sample of cells transfected with MDR6. As can be seen, similar to Lucentis, MDR5 and MDR6 can specifically bind to hVEGF, but do not bind to mouse VEGF and rat VEGF. Lucentis binds to human hVEGF165 and does not recognize mouse or rat VEGF. The antibodies expressed in the culture supernatant of cells transfected with MDR-5 and MDR-6 behave in the same manner as the standard Lucentis. They only bind to human hVEGF165 and do not recognize murine VEGF.

### Example 4: ELISA verification of characteristic binding of vector-expressed antiVEGF Fab

Fig. 3: To a highly adsorbing and binding 96-well plate, samples were added and plated in different columns respectively: column 1: human VEGF165 10 ng; column 2: mouse serum 10 ug; column 3: rat serum 10 ug; column 4: human serum No. 1 10 ug; column 5: human serum No. 2 10 ug; column 6: human serum No. 3 10 ug; column 7: mouse serum 10 uL (about 610 ug protein); column 8: rat serum 10 uL (about 680 ug protein); column 9: human serum No. 1 10 uL (about 820 ug protein); column 10: human serum No. 2 10 uL (about 720 ug protein); column 11: human serum No. 3 10 uL (770 ug protein). The samples were 2-fold diluted serially with a carbonate buffer and the plate was coated overnight at 4°C. According to the standard operation procedure of ELISA, 100 µL of 200 ng/mL Lucentis was added to each well and incubated at 37°C. Anti-Human Kappa-HRP antibody was used as the secondary antibody for incubation. The HRP substrate was added for development. Then the reaction was terminated, and the binding of Lucentis to VEGF in the serum samples was detected.

As shown in Fig. 3, VEGF in column 1 and human serum in columns 4-6 and 9-11 all have color reactions with Lucentis. Mouse serum in columns 2 and 7 and rat serum in columns 3 and 8 do not have color reactions with Lucentis. This suggests that Lucentis only recognizes human hVEGF165 and human serum samples, and does not recognize VEGF in rat and mouse serum samples.

### Example 5: Production of AAVMDR4, AA VMDR5 and AAVMDR6 with three-plasmid system

293T cells were co-transfected with pAdHelper, pAAV-RC, and pAAVMDR-NFS. The 293T cells were seeded in a cell factory and collected after 72 hrs. The cells were re-suspended in a cell lysis buffer and subjected to 3 cycles of freezing and thawing. The virus rAAVMDR described in the present application was separated, concentrated, and purified by ultracentrifugation, adsorption by QHP anion column and elution, and concentration.

SDS-PAGE separation gel and stacking gel were prepared. 15 µL of sample was added to each well. After electrophoresis, the sample was stained with Coomassie Brilliant Blue and destained with a conventional destaining solution for identification of virus purity.

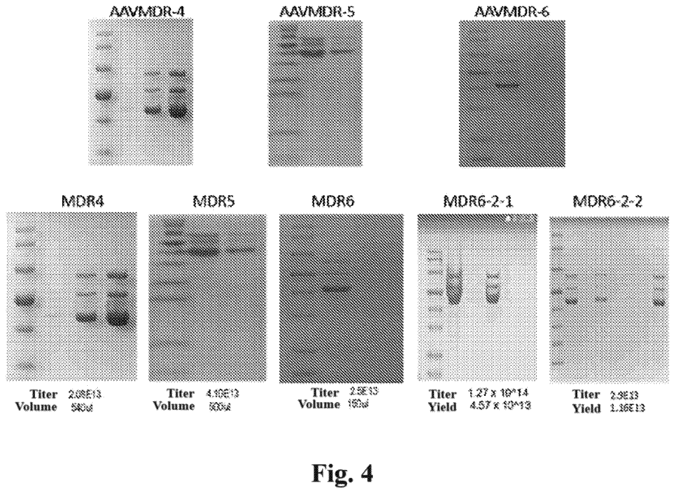

Fig. 4 shows the purity identification of purified AAVMDR4, AAVMDR5 and AAVMDR6 viruses, where Lane E is the purified virus sample. The electrophoregram shows that VP1/VP2/VP3=1:1:10, which is consistent with the ratio characteristics of AAV capsid proteins. The band is clear and the purity is above 90%. The physical titer of rAAVMDR virus was measured by fluorescence quantitative PCR. The genome titers of AAVMDR4, AAVMDR5 and AAVMDR6 samples were 2.08E13, 4.10E13 and 2.5E13 vg/mL respectively.

### Example 6: Expression and quantification of viruses in in-vitro studies

The expression of anti-VEGF antibodies was detected to compare the viral expression of MDR2, MDR4, MDR5 and MDR6. 293T cells were taken and cultured in a 6-well plate. After 48 hrs, a virus infection experiment was performed. The complete culture medium (DMEM, 10% FBS, 1% glutamine substitute (2 mM)) in the 6-well plate was discarded and 1 mL of fresh infection medium (DMEM, 1% glutamine substitute (2 mM)) was added. After the medium was changed, viral virions with MOI=1E5 was added to the 6-well plate, and incubated at 37°C for 1 hr. 2 mL of medium was supplemented, and the incubated was continued for 72 hrs. The cell culture supernatant was collected and concentrated and then subjected to WB and ELISA assay to detect the content of the anti-VEGF antibody. The purchased Lucentis injection was used as a standard.

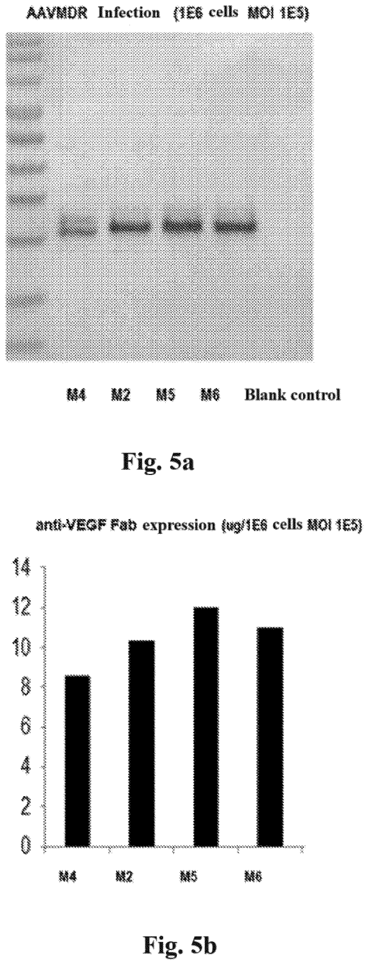

Fig. 5a shows the identification of anti-VEGF Fab expression in the supernatant samples of cells infected with viruses MDR4 (M4), MDR2 (M2), MDR5 (M5), and MDR6 (M6). From left to right: M4, M2, M5, M6 and blank control samples. Fig. 5b shows cells infected with virus M4, M2, M5, and M6: 1E6 cell, MOI=1E5. 72 hrs after infection, the supernatant was collected, and the anti-VEGF Fab content was measured by ELISA, with the Lucentis injection as a standard. From left to right: M4 (calculated content is 8.09 ug), M2 (calculated content is 10.17 ug), M5 (calculated content is 12.28 ug) and M6 (calculated content is 11.09 ug).

### Example 7: Viral expression in mouse eyes: Method: Mice: C57 mice, 6-8 wks (age)

Test group: The mice were injected with AAVMDR (4 × 10E 10vg); and control group: the mice were injected with PBS into the vitreous cavity. 4 weeks after injection, the injected eyeballs were removed, and 100 uL of RIPA lysis buffer was added to each eyeball. The tissue was homogenized, ultrasonicated, and centrifuged at 12,000 rpm for 2 min. The supernatant was taken, and the anti-VEGF Fab expression was detected by Western assay. The loading volumes are: 2.5 uL, and 5 uL respectively; positive control: Lucentis protein: 50 ng; negative control: eyeball sample injected with PBS. Detection: anti-human-K-HRP.

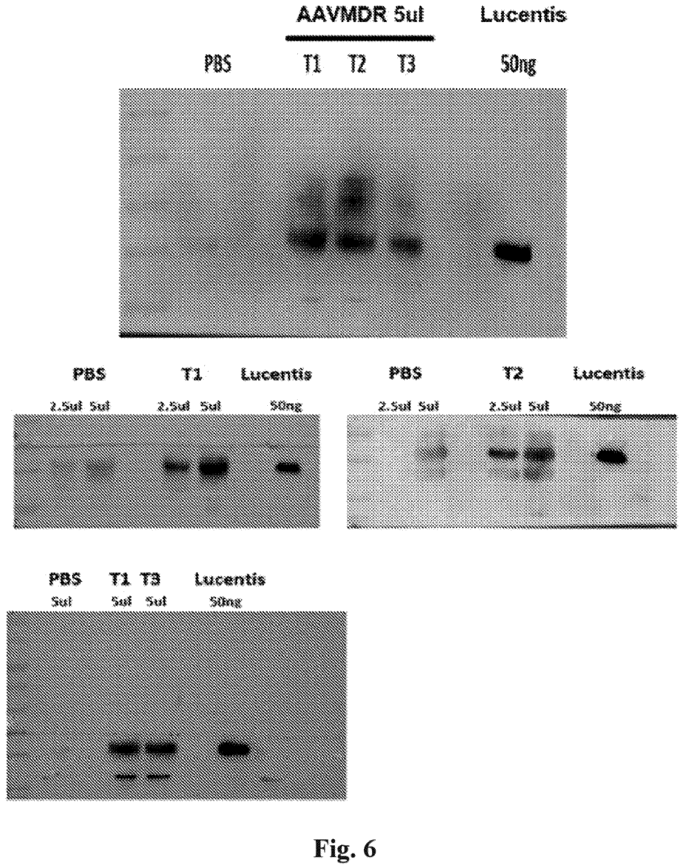

As shown in Fig. 6: A clear Fab expression band was detected in the eyeballs of mice injected with AAVMDR virus, and the molecular weight was consistent with the Lucentis standard.

### Example 8: Detection of anti-VEGF Fab expression (ng/eye) after intravitreal injection of AA VMDR5 into mice for two weeks

C57/BL6 mice were divided into a test group (rAAV-antiVEGF Fab virus group) and a control group (PBS group). 1 microliter of the virus AAVMDR described in the present application was injected into the vitreous cavity of the mice, and the doses were 5E9vg, 1E10vg, 2E10vg, and 4E10vg respectively. The mice in the blank group was injected with the same volume of PBS. The mouse eyeballs were removed after 2 weeks. A RIPA lysis buffer was added to the injected eyeball, and the tissue was homogenized and ultrasonicated. After centrifugation at 12,000 rpm for 2 min, the supernatant was collected, aliquoted and stored at -20°C.

The expression level in mouse eyeballs was detected by ELISA. To a highly adsorbing and binding 96-well plate, 50 ng of hVEGF was added to each well, and the plate was coated overnight at 4°C. The starting concentration of the standard Lucentis was 100 ng/mL, and 2-fold diluted serially. 2 µl of the injected eyeball homogenate supernatant of the test group or the blank group was added to each sample column, and 2 replicate wells were set and incubated at 37°C for 2 hrs. The antibody Anti-Human Kappa-HRP conjugated antibody was 1:4000 diluted, and 100 µL was added to each well, and incubated at room temperature for 1 hr. After washing, 100 µL of a TMB developing solution was added to each well, and incubated at room temperature in the dark for 20 min. Then 100 µL of a stop solution was added to each well. The absorbance at 450 nm was measured on a full-wavelength microplate reader, and the experimental data was fitted, regressed, and calculated by a four-parameter method. The calculated content from the experimental data was normalized to the data of the PBS control group.

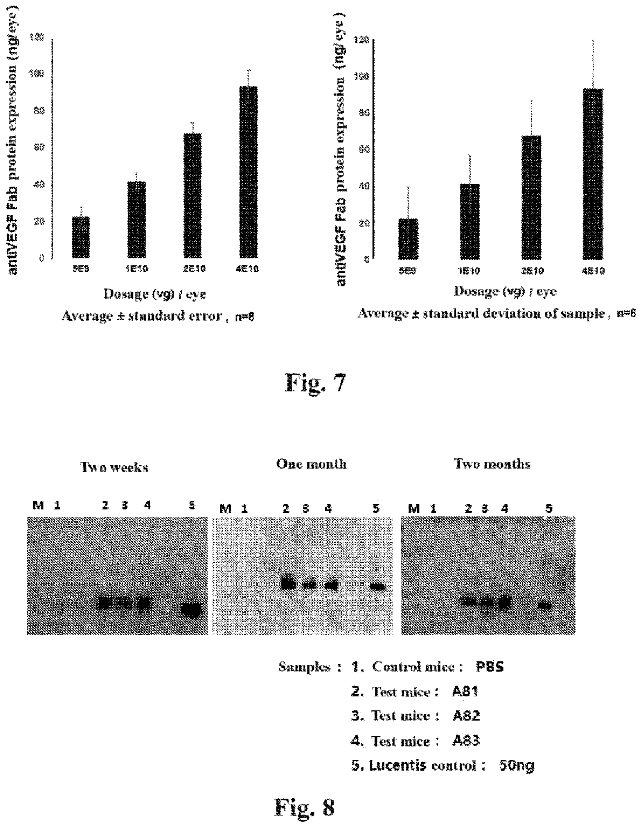

Fig. 7 shows, from left to right, the experimental data of the 5E9 sample, the 1E10 sample, the 2E10 sample and the 4E10 sample respectively. The expression level of anti-VEGF Fab in mouse eyeballs increases with increasing dose, and is thus consistent with a dose-dependent effect.

As shown in Fig. 8, after the C57 mice were injected with the virus AAVMDR5 described in the present application (at a dosage of 4E11vg), or the mice in the blank group was injected with the same volume of PBS, the anti-VEGF Fab in the mice in the virus group is continuously expressed over two months after the injection.

### Example 9: Research on anti-ocular vascular proliferation of AAVMDR5 virus

The human Rhodopsin promoter was used to drive the specific expression of human VEGF165 in the eyes of mice, resulting in the symptoms of ocular neovascularization. The degree of vascular proliferation was graded, and the degree of improvement of vascular proliferation by human derived anti-human VEGF antibodies was observed to study the biological effects of anti-human VEGF drugs. Qualified transgenic mice were selected through genotype identification and fundus observation of transgenetic mice, and injected with viruses to study their biological effects against ocular vascular proliferation. The transgenic mice were divided into a test group and a blank group. In the test group, 1 microliter of the virus AAVMDR5 described in the present application was injected into the vitreous cavity of the mice by a microsyringe at a dose of 1E10vg. The mice in the blank group was injected with the same volume of PBS. Anti-inflammatory eye drops were dripped every day for 7 days after the surgery, and the eyes of the mice were observed every day.

Two weeks after the injection of the virus AAVMDR5 according to the present application, the mice were anesthetized by intraperitoneal injection with 5% chloral hydrate, and about 2 ml of normal saline and then 1 ml of physiological saline containing 50 mg dextran-FITC (molecular weight: 2 × 10⁶) were perfused into the systemic circulation through the left ventricle. The mice were sacrificed by breaking the spine, and the eyeballs were removed, rinsed with PBS, and immobilized with 4% paraformaldehyde for 20 min. The sclera was cut from the posterior portion of the ciliary body near the ora serrata, and the anterior segment and the vitreous body were removed. The retina was peeled off under a microscope, cut into four petals, transferred to 4% paraformaldehyde and immobilized. The sample was rinsed three times with PBS. The retinal ganglion cell layer was laid upward on a glass slide, an anti-fluorescence quenching mounting medium was added, and the slide was covered with a coverslip. The samples were observed under a fluorescence microscope and photographed.

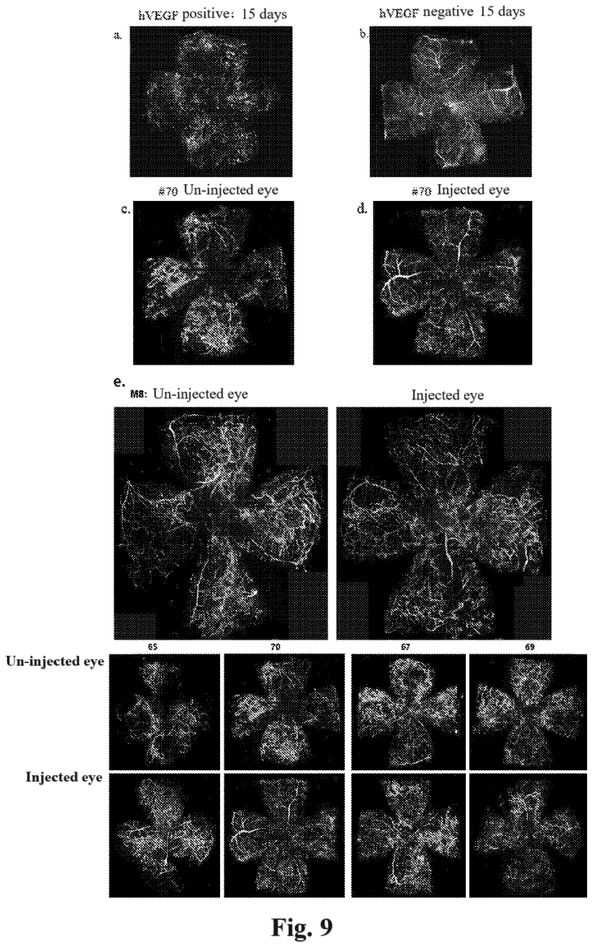

Fig. 9 shows the images of retina preparations after Dextran-FITC perfusion into mice: (a): fluorescence image of retinal blood vessels in hVEGF positive transgenic mouse strain TO36939 at day 15; (b): fluorescence image of retinal blood vessels in hVEGF negative mouse strain TO36939 at day 15; (c): fluorescence image of retinal blood vessels in hVEGF positive mice strain TO36939 2 weeks after intravitreal injection of PBS (age: 40 days); (d): fluorescence image of retinal blood vessels in hVEGF positive mice strain TO36939 2 weeks after intravitreal injection of AAVMDR5 virus (age: 40 days); and (e): comparison of fluorescence images of retinal blood vessels in the vitreous cavity of uninjected eyes and injected eyes (AAVMDR5 virus, 1E10 vg/eye) of hVEGF-positive mice strain TO36939 2 weeks after injection (age: 40 days). It can be seen from the image that at day 15, the number of neovascular sprouts in the retina of hVEGF positive mice increases significantly. The retinal tissue of the negative mice was intact, the blood vessels were clear, and there are no signs of neovascularization. Two weeks after administration, the uninjected eyes of hVEGF-positive mice show severe symptoms of vascular proliferation, and the fluorescence intensity shows disordered expansion of blood vessels; and the eyes of mice injected with AAVMDR5 virus have weakened symptoms of retinal neovascularization, and the fluorescence intensity is significantly weakened.

### Example 10: Cell transfection, expression and screening of viral vector plasmids after codon optimization

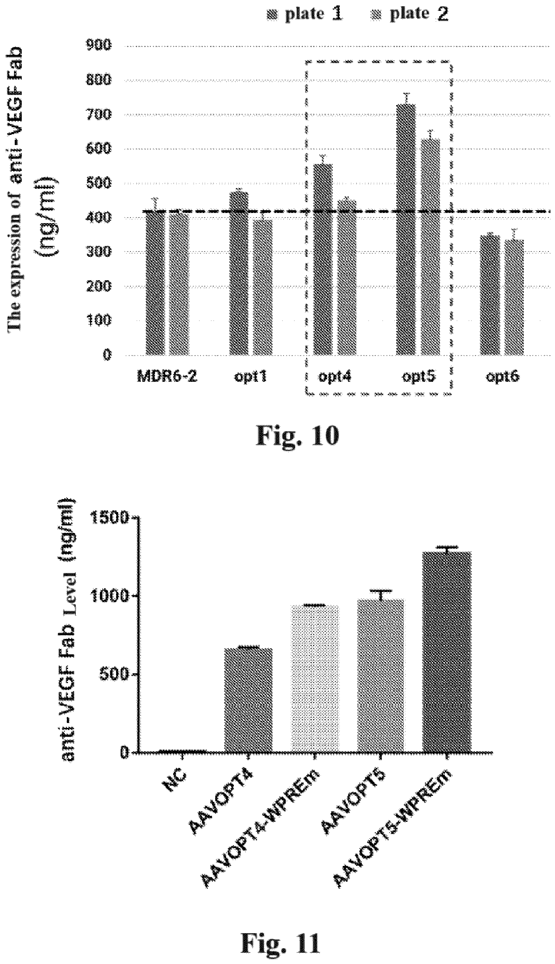

The MDR6, OPT1, OPT4, OPT5, and OPT6 sequences as described above were constructed into the pcDNA3.1 vector respectively. The heavy chain nucleotide sequences of MDR6-2, OPT1, OPT4, OPT5, and OPT6 are SEQ ID NO: 5, SEQ ID NO: 23, SEQ ID NO: 24, SEQ ID NO: 25, and SEQ ID NO: 26, respectively. The light chain nucleotide sequences are SEQ ID NO: 4, SEQ ID NO: 19, SEQ ID NO: 20, SEQ ID NO: 21, and SEQ ID NO: 22 respectively. The HEK293 cells were grown to a density of 90% or more. The cells were digested and collected, and inoculated into a 6-well plate at a density of 1 × 10⁶. 6 samples were transfected respectively (with the above 6 plasmids). Each sample was duplicated. 2.5 ug of each plasmid and PEI were used for transfection. The plasmid:PEI ratio was 1:3. 72 hrs after transfection, the cell culture supernatant was collected for ELISA detection. As shown in Fig. 10, the samples from left to right are MDR6-2 (the average of the two wells is 414 ng/ml), OPT1 (the average of the two wells is 434 ng/ml), OPT4 (the average of the two wells is 503 ng/ml), OPT5 (the average of the two wells is 678 ng/ml) and OPT6 (the average of the two wells is 342 ng/ml), among which OPT4 and OPT5 have the highest expression levels.

The AAVOPT4, AAVOPT4-WPREm, AAVOPT5, and AAVOPT5-WPREm plasmids were further constructed (the "WPREm" in the plasmid name indicates that the expression vector contains a WPREm element (SEQ ID NO: 27)). The HEK293 cells were grown to a density of 90% or more. The cells were digested and collected, and inoculated into a 12-well plate at a density of 3 × 10⁵. 4 samples were transfected respectively (with the above 4 plasmids). 1.25 ug of each plasmid and PEI were used for transfection. The plasmid:PEI ratio was 1:3. The same amount of PBS was added to the blank group. 72 hrs after transfection, the cell culture supernatant was collected for ELISA detection. The results are shown in Fig. 11. From left to right, they are the blank group (NC), AAVOPT4, AAVOPT4-WPREm, AAVOPT5 and AAVOPT5-WPREm. The expression levels are 5 ng/ml, 657 ng/ml, 929 ng/ ml, 970 ng/ml and 1268 ng/ml respectively. The expression level increases significantly after WPREm is added, and the expression level of AAVOPT5-WPREm is the highest.

### Example 11: Production of AAVOPT5-WPREm with three-plasmid system

293T cells were co-transfected with pAdHelper, pAAV-RC, and pAAV- MDR-NFS. The 293T cells were seeded in a cell factory and collected after 72 hrs. The cells were re-suspended in a cell lysis buffer. The virus rAAVOPT5-WPREm described in the present application was separated, concentrated, and purified by ultracentrifugation, adsorption by QHP anion column and elution, and concentration. After the virus was lyzed, the "virus concentrate" was 1000-fold, 10000-fold and 100000-fold serially diluted to detect the virus titer by QPCR.

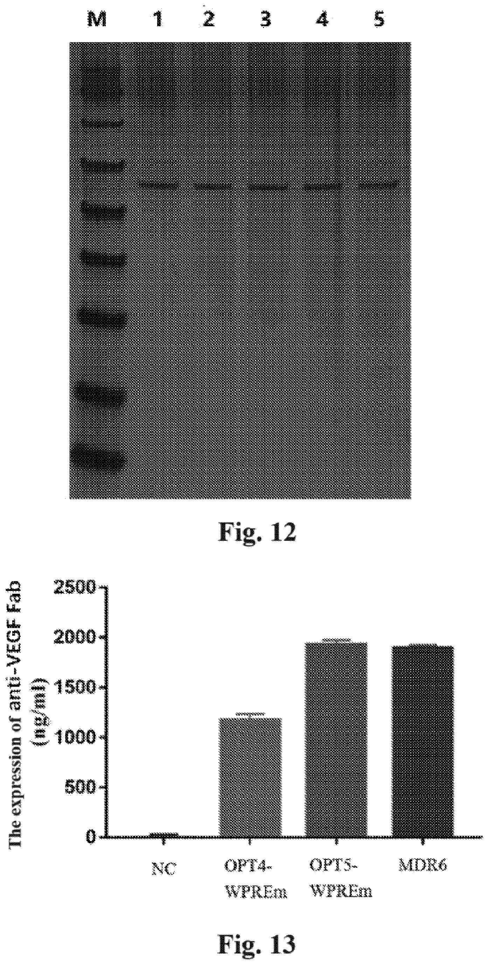

SDS-PAGE separation gel and stacking gel were prepared. 2E10vg/well of sample was added to each well. After electrophoresis, the sample was stained with Coomassie Brilliant Blue and destained with a conventional destaining solution for identification of virus purity. Fig. 12 shows the purity identification of purified AAVOPT5-WPREm virus, in which M is pre-stained protein Marker (Abs924) 0.5 ul, 1 and 2 are AAVMDR6 2 × 10¹⁰, 3 and 4 are AAVOPT5-WPREm 2 × 10¹⁰, and 5 is AAV2- IRES-hrGFP 2 × 10¹⁰. The electrophoregram shows that VP1/VP2/VP3=1:1:10, which is consistent with the ratio characteristics of AAV capsid proteins. The band is clear and the purity is above 90%.

### Example 12: Expression and screening of virus-infected cells

Anti-VEGF Fab expression in the supernatant samples after the cells were infected with MDR6, OPT4-WPREm, and OPT5-WPREm were identified. 1 × 10⁵ HEK293 cells were inoculated in a 12-well plate, and then infected with MDR6 and OPT5, with MOI=1E5. 72 hrs after infection, the supernatant was collected, and the anti-VEGF Fab content was measured by ELISA, with the Lucentis injection as a standard.

As shown in Fig. 13, the expression level of OPT4-WPREm is 1173 ng/ml, the expression level of OPT5-WPREm is 1927 ng/ml, and the expression level of MDR6-2 is 1893 ng/ml, among which OPT5 has the highest expression level.

### Example 13: Study on the effect of AAVOPT5-WPREm virus on the proliferation of HUVECs

The proliferation efficiency of HUVECs cells is affected by the VEGF content. The proliferation rate of HUVECs is used as an indicator to determine the changes in VEGF content, thereby verifying the mechanism of action of the AAVOPT5 drug.

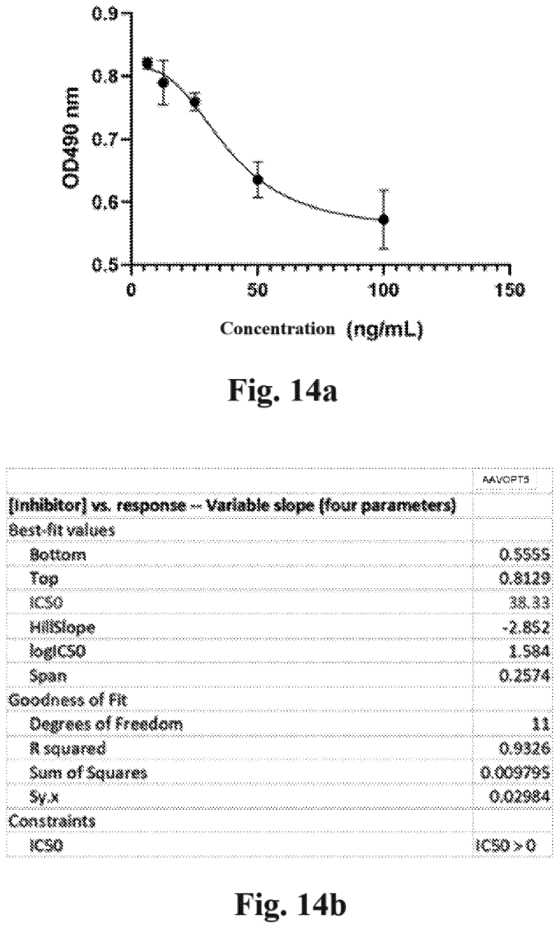

As shown in Fig. 14a, the proliferation efficiency of HUVECs is inversely related to the dose of AAVOPT5-WPREm drug, indicating that AAVOPT5 blocks the activity of hVEGFA to prevent the proliferation of HUVECs, and the blocking effect increases as the dose increases. As shown in Fig. 14b, the half inhibitory concentration (IC50) for 10 ng/mL hVEGFA is 38.33 ng/mL as calculated by Graphpad.

### Example 14: AAVOPT5-WPREm has anti-angiogenic effect

HUVECs will form a tube structure on Matrigel under the guidance of VEGFA. When VEGFA is blocked, the tube formation of HUVECs is blocked. A basement membrane extract (BME, R&D systems) with reduced growth factor was added to a 96-well plate and allowed to stand for 30 min. HUVEC (5 × 10 ³ cells/well) was suspended in a basal medium containing VEGF-A (25 ng/ml) and placed in the 96-well plate to which BME had been added in advance. The cells were treated with 1 ng, 5 ng, and 10 ng AAVOPT5-WPREm respectively. After incubation for 16 hrs, the tube formation was observed and imaged. The area of the tube network and the tube length were statistically counted by angiogenesis analysis plug-in of ImageJ software (with pixels as the unit of measurement).

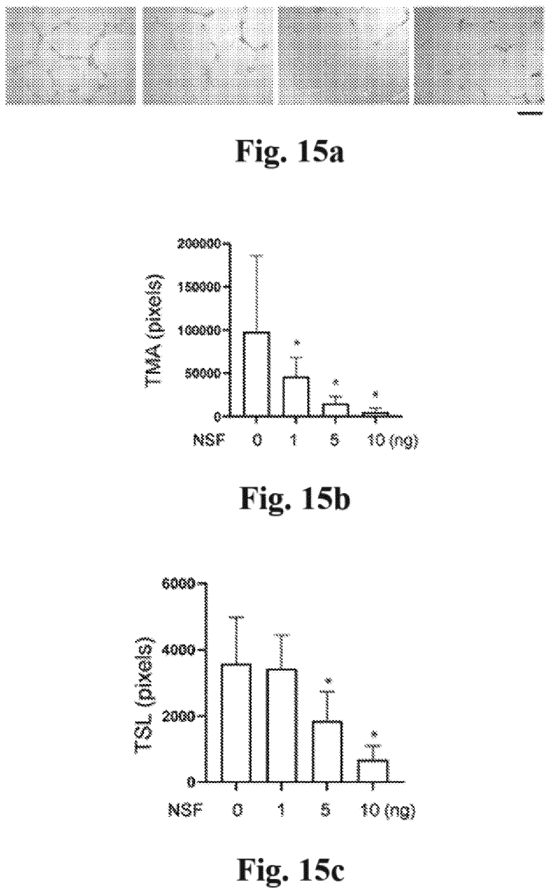

Fig. 15a-15c compare the tube formation of HUVECs without/with the addition of various amounts of AAVOPT5-WPREm. Images in Fig. 15a (from left to right: no addition, addition of 1 ng, 5 ng, and 10 ng) and the statistical graphs of tube area and tube length (Figs. 15b and 15c) show that the tube area (TMA) and tube length (TSL) decrease significantly with the increase of AAVOPT5 drug concentration. Where AAVOPT5-WPREm is not added, the tube area is about 100000 pixels, and the tube length is about 3600 pixels. When 1 ng AAVOPT5 is added, the tube area is about 48000 pixels, and the tube length is about 3500 pixels. When 5 ng AAVOPT5-WPREm is added, the tube area is about 10,000 pixels, and the tube length is about 1,800 pixels. When 10 ng AAVOPT5-WPREm is added, the tube area is about 2,000 pixels, and the tube length is about 600 pixels.

### Example 15: Study on the effect of AAVOPT5-WPREm virus against the ocular vascular proliferation

The human Rhodopsin promoter was used to drive the specific expression of human VEGF165 in the eyes of mice, resulting in the symptoms of ocular neovascularization. The degree of vascular proliferation was graded, and the degree of improvement of vascular proliferation by human derived anti-human VEGF antibodies was observed to study the biological effects of anti-human VEGF drugs. Qualified transgenic mice were selected through genotype identification and fundus observation of transgenetic mice, and injected with viruses to study their biological effects against ocular vascular proliferation. The transgenic mice were divided into a test group and a blank group. In the test group, 1.5 microliter of the virus AAVOPT5 described in the present application was injected into the vitreous cavity of the mice by a microsyringe at a dose of 1E10vg. The mice in the blank group was injected with the same volume of PBS. Anti-inflammatory eye drops were dripped every day for 7 days after the surgery, and the eyes of the mice were observed every day.

28 days after the injection of the virus AAVOPT5-WPREm, 2 mg/ml Doxycycline was used to induce the expression of human VEGF165 in the eyes of the treated mice, and symptoms of ocular vascular proliferation appeared. 7 days after induction, the mice were anesthetized by intraperitoneal injection with 5% chloral hydrate, and about 2 ml of normal saline and then 1 ml of physiological saline containing 50 mg dextran-FITC (molecular weight: 2 × 10⁶) were perfused into the systemic circulation through the left ventricle. The mice were sacrificed by breaking the spine, and the eyeballs were removed, rinsed with PBS, and immobilized with 4% paraformaldehyde for 20 min. The sclera was cut from the posterior portion of the ciliary body near the ora serrata, and the anterior segment and the vitreous body were removed. The retina was peeled off under a microscope, cut into four petals, transferred to 4% paraformaldehyde and immobilized. The sample was rinsed three times with PBS. The retinal ganglion cell layer was laid upward on a glass slide, an anti-fluorescence quenching mounting medium was added, and the slide was covered with a coverslip. The samples were observed under a fluorescence microscope and photographed.

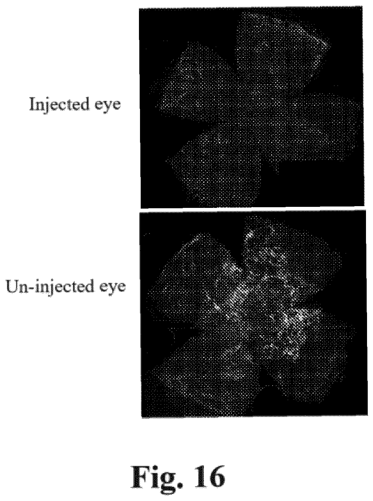

Fig. 16 shows images of the retina preparations after Dextran-FITC perfusion in mice, and compares fluorescence images of retinal blood vessels 7 days after Doxycycline induction and 35 days after injection in the vitreal cavities in un-injected eyes and injected eyes of hVEGF-positive T03943 mice (AAVOPT5-WPREm virus, 1E10 vg/eye). As can be seen, the uninjected eyes of hVEGF-positive mice show severe symptoms of vascular proliferation, and the fluorescence intensity shows disordered expansion of blood vessels; and the eyes of mice injected with AAVOPT5-WPREm virus have weakened symptoms of retinal neovascularization, and the fluorescence intensity is significantly weakened.

## Claims

1. A recombinant nucleic acid, encoding an anti-VEGF heavy chain, the coding sequence of the anti-VEGF heavy chain comprising a sequence having ≥90%, ≥95%, ≥96%, ≥97%, ≥98%, ≥99%, ≥99.5%, or 100% identity to one of the sequences as shown in SEQ ID NOs: 5-6 and 23-26.

2. The recombinant nucleic acid according to claim 1, wherein the amino acid sequence of the anti-VEGF heavy chain comprises a sequence having ≥90%, ≥95%, ≥96%, ≥97%, ≥98%, ≥99%, ≥99.5% or 100% identity to one of the sequences as shown in SEQ ID NOs: 17 and 18.

3. The recombinant nucleic acid according to claim 1 or 2, wherein the sequences of the complementarity determining region 1 (CDR1), the complementarity determining region 2 (CDR2), and the complementarity determining region 3 (CDR3) of the anti-VEGF heavy chain are identical to the sequences as shown in SEQ ID NO: 31, SEQ ID NO: 32, and SEQ ID NO: 33, respectively.

4. The recombinant nucleic acid according to any one of claims 1 to 3, wherein the anti-VEGF heavy chain does not include a constant region 2 (CH2) and a constant region 3 (CH3).

5. A recombinant nucleic acid, encoding an anti-VEGF light chain, the coding sequence of the anti-VEGF light chain comprising a sequence having ≥90%, ≥95%, ≥96%, ≥97%, ≥98%, ≥99%, ≥99.5% or 100% identity to one of the sequences as shown in SEQ ID NOs: 4 and 19 to 22.

6. The recombinant nucleic acid according to claim 5, wherein the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO: 16.

7. The recombinant nucleic acid according to claim 5 or 6, wherein the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30 respectively.

8. The recombinant nucleic acid according to any one of claims 1 to 4, wherein the recombinant nucleic acid further encodes an anti-VEGF light chain, and the anti-VEGF light chain encoding sequence comprises a sequence having ≥90%, ≥95%, ≥96%, ≥97%, ≥98%, ≥99%, ≥99.5% or 100% identity to one of the sequences as shown in SEQ ID NOs: 4 and 19 to 22.

9. The recombinant nucleic acid according to claim 8, wherein the amino acid sequence of the anti-VEGF light chain comprises a sequence having ≥90%, ≥95%, ≥96%, ≥97%, ≥98%, ≥99%, ≥99.5% or 100% identity to the sequence as shown in SEQ ID NO: 16 .

10. The recombinant nucleic acid according to claim 9, wherein the sequences of the complementarity determining region 1 (CDR1), the complementarity determining region 2 (CDR2), and the complementarity determining region 3 (CDR3) of the anti-VEGF light chain are identical to the sequences as shown in SEQ ID NO: 28, SEQ ID NO: 29, and SEQ ID NO: 30 respectively.

11. The recombinant nucleic acid according to any one of claims 8 to 10, wherein the combination of SEQ ID NOs: 4 and 19 to 22 with SEQ ID NOs: 5 and 6 and 23 to 26 is a combination selected from Table 1.

12. The recombinant nucleic acid according to any one of claims 8 to 10, wherein the combination of SEQ ID NOs: 4 and 19 to 22 with SEQ ID NOs: 5 and 6 and 23 to 26 is a combination selected from Table 3.

13. The recombinant nucleic acid according to any one of claims 8 to 10, wherein the coding sequence of the anti-VEGF heavy chain comprises a sequence having ≥90%, ≥95%, ≥96%, ≥97%, ≥98%, ≥99%, ≥99.5% or 100% identity to the sequence as shown in SEQ ID NO: 25, and the coding sequence of the anti-VEGF light chain comprises a sequence having ≥90%, ≥95%, ≥96, ≥97%, ≥98%, ≥99%, ≥99.5% or 100% identity to the sequence as shown in SEQ ID NO: 21.

14. The recombinant nucleic acid according to any one of claims 8 to 10, wherein the coding sequence of the anti-VEGF heavy chain comprises a sequence having ≥99%, ≥99.5% or 100% identity to the sequence as shown in SEQ ID NO: 25, and the coding sequence of the anti-VEGF light chain comprises a sequence having ≥99%, ≥99.5% or 100% identity to the sequence as shown in SEQ ID NO: 21.

15. The recombinant nucleic acid according to any one of claims 8 to 14, wherein the coding sequence of the anti-VEGF light chain is located upstream (at 5' end) of the coding sequence of the anti-VEGF heavy chain.

16. The recombinant nucleic acid according to any one of claims 8-15, further comprising a coding sequence of a linker peptide between the coding sequence of the anti-VEGF light chain and the coding sequence of the anti-VEGF heavy chain.

17. The recombinant nucleic acid according to claim 16, wherein the amino acid sequence of the linker peptide comprises any one of the sequences as shown in SEQ ID NOs: 14-15, or a sequence that differs from any one of the sequences as shown in SEQ ID NOs: 14-15 by no more than 1, no more than 2, or no more than 3 amino acids.

18. The recombinant nucleic acid according to any one of claims 1 to 17, further comprising a promoter and/or an enhancer, wherein the promoter and/or the enhancer is operably linked to an open reading frame encoding the anti-VEGF light chain and/or the anti-VEGF heavy chain; optionally, the promoter is a chicken β-actin promoter; and optionally, the enhancer is a CMV enhancer.

19. The recombinant nucleic acid according to claim 18, wherein the sequence of the promoter/enhancer comprises a sequence having ≥90%, ≥95%, ≥96%, ≥97%, ≥98%, ≥99%, ≥99.5% or 100% identity to the sequence as shown in SEQ ID NO:1.

20. The recombinant nucleic acid according to any one of claims 1 to 19, further comprising one or more signal peptide coding sequences, wherein the signal peptide coding sequence is located upstream (at 5' end) of the coding sequence of the anti-VEGF heavy chain and/or the coding sequence of the anti-VEGF light chain.

21. The recombinant nucleic acid according to claim 20, wherein the signal peptide coding sequence is connected to the 5' end of the coding sequence of the anti-VEGF heavy chain; and preferably, the signal peptide coding sequence connected to the anti-VEGF heavy chain comprises an amino acid sequence that is ≥90%, ≥95%, ≥96%, ≥97%, ≥98%, ≥99%, or 100% identical to any one of the sequences as shown in SEQ ID NOs: 8-13 and 34 and 35, and preferably ≥90%, ≥95%, ≥96%, ≥97%, ≥98%, ≥99%, or 100% identical to the sequence as shown in SEQ ID NO: 34.

22. The recombinant nucleic acid according to claim 20 or 21, wherein the signal peptide coding sequence is connected to the 5' end of the anti-VEGF light chain; and preferably the signal peptide coding sequence connected to the anti-VEGF light chain comprises an amino acid sequence that is ≥90%, ≥95%, ≥96%, ≥97%, ≥98%, ≥99%, or 100% identical to any one of the sequence as shown in SEQ ID NOs: 8-13 and 34 and 35, and preferably ≥90%, ≥95%, ≥96%, ≥97%, ≥98%, ≥99%, or 100% identical to the sequence as shown in SEQ ID NO: 35.

23. The recombinant nucleic acid according to any one of claims 1 to 22, further comprising an intron, wherein optionally, the intron is located between the sequence of the promoter/enhancer and the signal peptide coding sequence.

24. The recombinant nucleic acid according to claim 23, wherein the sequence of the intron comprises a sequence that is ≥90%, ≥95%, ≥96%, ≥97%, ≥98%, ≥99%, ≥99.5% or 100% identical to any one of the sequence as shown in SEQ ID NO: 2 and 3 .

25. The recombinant nucleic acid according to any one of claims 1 to 24, further comprising a poly(A) sequence, wherein the poly(A) sequence is located downstream (at 3' end) of the coding sequence of the anti-VEGF heavy chain and/or the coding sequence of the anti-VEGF light chain; and optionally, the poly(A) sequence is human growth hormone poly(A) sequence.

26. The recombinant nucleic acid according to claim 25, wherein the poly(A) sequence comprises a sequence that is ≥90%, ≥95%, ≥96%, ≥97%, ≥98%, ≥99%, ≥99.5%, or 100% identical to the sequence as shown in SEQ ID NO: 7.

27. The recombinant nucleic acid according to any one of claims 1 to 26, further comprising a woodchuck hepatitis virus post-transcriptional regulatory element (WPRE) sequence, wherein the WPRE sequence is located between the open reading frame encoding the anti-VEGF light chain and/or the anti-VEGF heavy chain and the poly(A) sequence.

28. The recombinant nucleic acid according to claim 27, wherein the WPRE sequence comprises a sequence that is ≥90%, ≥95%, ≥96%, ≥97%, ≥98%, ≥99%, ≥99.5% or 100% identical to the sequence as shown in SEQ ID NO: 27.

29. A viral vector, comprising the recombinant nucleic acid according to any one of claims 1 to 28.

30. The viral vector according to claim 29, wherein the vector is an adeno-associated virus (AAV) vector.

31. The viral vector according to claim 30, wherein the serotype of the adeno-associated virus vector is one or more selected from the group consisting of AAV1, AAV2, AAV3, AAV4, AAV5, AAV6, AAV7, AAV8, AAV9, AAVrh10, AAV2.7m8, AAV- DJ, AAV-PHP.B, AAV-PHP.S, and AAV-PHP.eB.

32. The viral vector according to claim 30 or 31, further comprising an inverted terminal repeat (ITR) of AAV.

33. A viral virion, comprising the viral vector according to any one of claims 29 to 32.

34. The viral virion according to claim 33, wherein the viral virion is an AAV viral virion.

35. The viral virion according to claim 34, wherein the serotype of the AAV virion is one or more selected from the group consisting of AAV1, AAV2, AAV3, AAV4, AAV5, AAV6, AAV7, AAV8, AAV9 , AAVrh10, AAV2.7m8, AAV-DJ, AAV-PHP.B, AAV-PHP.S, and AAV-PHP.eB.

36. A pharmaceutical preparation, comprising the viral virion according to any one of claims 33 to 35, the viral vector according to any one of claims 29 to 32, or the recombinant nucleic acid according to any one of claims 1 to 28, and a pharmaceutically acceptable carrier or excipient.

37. The pharmaceutical preparation according to claim 36, wherein the pharmaceutical preparation is a liquid preparation.

38. Use of the viral virion according to any one of claims 33 to 35, the viral vector according to any one of claims 29 to 32, or the recombinant nucleic acid according to any one of claims 1 to 28 in the preparation of medicines for treating eye diseases.

39. A method for treating eye diseases, comprising administering to a subject in need thereof the pharmaceutical preparation according to any one of claims 36 and 37, the viral virion according to any one of claims 33 to 35, the viral vector according to any one of claims 29 to 32, or the recombinant nucleic acid according to any one of claims 1 to 28.

40. The use according to claim 38 or the method according to claim 39, wherein the eye disease is choroidal neovascular disease.

41. The use or method according to claim 40, wherein the choroidal neovascular disease is age-related macular degeneration or diabetic retinal degeneration; and preferably, the diabetic retinal degeneration is diabetic macular edema.

42. The use or method according to any one of claims 38 to 41, wherein the viral virion, the viral vector, the recombinant nucleic acid, or the pharmaceutical preparation is injected intraocularly, and preferably, intravitreally injected.

43. The use or method according to any one of claims 38 to 42, wherein the viral virion, the viral vector, or the pharmaceutical preparation are capable of long-lasting expression of the anti-VEGF antibody or an antigen binding fragment thereof in the eye, to prevent the eye diseases caused by VEGF overexpression.
